(19) **Europäisches Patentamt**
**European Patent Office**
**Office européen des brevets**

(11) **EP 1 685 831 B1**

(12) **EUROPEAN PATENT SPECIFICATION**

(45) Date of publication and mention
of the grant of the patent:
**07.03.2007 Bulletin 2007/10**

(51) Int Cl.:
***A61K 31/137*** *(2006.01)*  ***A61K 9/22*** *(2006.01)*

(21) Application number: **05000985.1**

(22) Date of filing: **19.01.2005**

(54) **Pharmaceutical polymer composition for oral controlled release delivery of terbutaline sulfate**

Pharmazeutische Polymerformulierung zur retardierten Freisetzung von Terbutalinsulfat

Composition pharmaceutique à base des polymers pour la libération prolongée de terbutaline sulphate

(84) Designated Contracting States:
**AT BE BG CH CY CZ DE DK EE ES FI FR GB GR HU IE IS IT LI LT LU MC NL PL PT RO SE SI SK TR**

(43) Date of publication of application:
**02.08.2006 Bulletin 2006/31**

(73) Proprietor: **The Jordanian Pharmaceutical Manufacturing Co.**
**Ltd.**
**11710 Naor (JO)**

(72) Inventors:
• **Badwan, Adnan**
**11185 Amman (JO)**
• **Al-Remawi, Mayyas**
**13713 Russiefa (JO)**

(74) Representative: **Winkler, Andreas Fritz Ernst**
**FORRESTER & BOEHMERT**
**Pettenkoferstrasse 20-22**
**80336 München (DE)**

(56) References cited:
**US-A- 4 814 176**   **US-A- 5 620 706**

• **MESHALI M M ET AL: "EFFECT OF INTERPOLYMER COMPLEX FORMATION OF CHITOSAN WITH PECTIN OF ACACIA ON THE RELEASE BEHAVIOR OF CHLORPROMAZINE HCL" INTERNATIONAL JOURNAL OF PHARMACEUTICS, AMSTERDAM, NL, vol. 89, no. 3, 1993, pages 177-181, XP009026688 ISSN: 0378-5173**
• **MELIA C D: "HYDROPHILIC MATRIX SUSTAINED RELEASE SYSTEMS BASED ON POLYSACCHARIDE CARRIERS" CRITICAL REVIEWS IN THERAPEUTIC DRUG CARRIER SYSTEMS, XX, XX, vol. 8, no. 4, 1991, pages 395-421, XP009026703 ISSN: 0743-4863**

## Description

The present invention is directed to a controlled-release pharmaceutical composition providing a sustained delivery of the basic drug Terbutaline sulfate, said composition comprising a hydrophilic polysaccharide polymer mixture of chitosan and xanthan gum and optionally comprising sodium bicarbonate.

## Background of the invention

Polymers that hydrate in an aqueous medium and form a gel structure are known as hydrophilic polymers such as chitosan and xanthan gum. These polymers are either cationic (as chitosan) or anionic (as xanthan) in chemical nature. Binary mixture of these polymers will lead to the formation of a gel layer at the surface of the tablet resulting in slow release of the active material out of the tablet. As a consequence, a prolongation of drug release is achieved.

When a solid dosage form comes into contact with acidic medium, as found in the stomach, for example, the hydratable polymers swell to form a gel. Sodium bicarbonate acts as a buffering agent that keeps the tablet micro-environment at pH 6-7. A cross-linking reaction occurs between cationic chitosan and anionic xanthan gum at this pH range. Thus, a layer of complex coacervate (hydrogel layer) is formed at the surface of the tablet. The drug to be released out of the tablet has to cross this hydrogel layer. The hydrogel barrier slows down the drug flux out of the tablet resulting in release retardation. Another mechanism for drug release retardation is provided by sodium bicarbonate. Sodium bicarbonate releases carbon dioxide ($CO_2$) gas inside the gel layer. The tablet density becomes low and a floating tablet is formed. $CO_2$ bubbles attached to the gel act as a barrier for drug release. Another advantage of $CO_2$ gas formation is the generation of a buoyant system. This system increases tablet resident time inside the gastrointestinal tract.

As described earlier in the European patent application No. 03019532.5, a ratio of 1:1 of chitosan and xanthan gum has been found a suitable universal controlled-release composition for controlling the release of basic drugs with a drug to polymer mixture ratio of 1:3 to 1:10.

US 4814176 describes a sustained-release tablet formulation on the basis of chitin and/or chitosan and an anionic polymer like polyacrylic acid or pectin. Terbutaline sulfate can be used as active agent.

Terbutaline sulfate is one example of a water-soluble basic drug. The solubility in water is >20 mg/ml, and the dissociation constant ($pK_a$) values have been reported to be 8.8, 10.1 and 11.2. The value of $pK_a$ = 10.1 can be assigned to the amino group. The other two $pK_a$ values, 8.8 and 11.2, may be attributed to the aromatic hydroxyl groups (S. Ahuja and J. Ashman, Terbutaline Sulfate. In: Analytical Profiles of Drug Substances, K. Florey (ed.), Vol. 19, Academic Press, 1990, pp. 603-625). Herein, Terbutaline sulfate was selected as a model drug.

Terbutaline sulfate is a beta-2 adrenergic agonist that is used as a bronchodilator. By relaxing bronchial tubes, Terbutaline prevents and treat wheezing, shortness of breath, and troubled breathing caused by asthma. It also is thought to relax the uterus' muscles, and thus, its use in preterm labor has been suggested. Terbutaline sulfate is typically prescribed in the pill form (2.5 or 5 mg pills at 3- 4- or 6-hour intervals), is administered by subcutaneous injection (e.g. by a pump with dosages of at least 3 mg per 24-hours) or is received by aerosol inhalation. The latter administration route takes the quickest therapeutic effect. However, in some indications it is preferred that the drug is delivered more steadily. When administered orally, Terbutaline sulfate has a rather short biological half-life (about 3.6 hrs). To overcome this disadvantage, its formulation in a controlled-release system would be of advantage (S. Ahuja and J. Ashman, supra).

Therefore, it is an object of the present invention to provide for a controlled-release pharmaceutical composition formulated for oral controlled-release delivery of the basic drug Terbutaline sulfate.

## Summary of the invention

The object of the present invention is solved by a controlled-release pharmaceutical composition comprising at least Terbutaline sulfate or a derivative thereof as an active agent, and further comprising an inactive matrix, said matrix comprising a hydrophilic polysaccharide polymer mixture, said mixture comprising chitosan or a derivative thereof, and further comprising xanthan gum or a derivative thereof, wherein the ratio of xanthan gum and chitosan within said mixture is in the range from about 1:10 to about 10:1.

In one embodiment, the ratio of xanthan gum and chitosan is in the range from about 1:2 to about 2:1.

In a preferred embodiment, the ratio of xanthan gum and chitosan is 1:1.

In one embodiment, the ratio of Terbutaline sulfate and the hydrophilic polysaccharide polymer mixture is in the range from about 1:1 to about 1:5.

In a preferred embodiment, the ratio of Terbutaline sulfate and the hydrophilic polysaccharide polymer mixture is 1:3.

In one embodiment, the controlled-release pharmaceutical composition optionally comprises at least one additional pharmaceutically acceptable filler.

In a preferred embodiment, the pharmaceutically acceptable filler is selected from the group comprising calcium hydrogen phosphate, mannitol, Avicel PH 101, sodium bicarbonate and the like.

In another preferred embodiment, the pharmaceutically acceptable filler is a carbon dioxide generating and/or pH controlling filler.

In a particular preferred embodiment, the pharmaceutically acceptable filler is sodium bicarbonate.

In one embodiment, the percentage of the pharmaceutically acceptable filler is in the range from about 5% to about 20% by total weight.

In a preferred embodiment, the percentage of the pharmaceutically acceptable filler is 12.5% by total weight.

In one embodiment, the percentage of the pharmaceutically acceptable filler together with the hydrophilic polysaccharide polymer mixture is in the range from about 70 to about 90% by total weight.

In a preferred embodiment, the pharmaceutically acceptable filler together with the hydrophilic polysaccharide polymer mixture is 83 % by total weight.

In one embodiment, the ratio of Terbutaline sulfate and the pharmaceutically acceptable filler on the one hand and the hydrophilic polysaccharide mixture on the other hand is in the range of about 1:1 to about 1:5.

In a preferred embodiment, the ratio of Terbutaline sulfate and the pharmaceutically acceptable filler on the one hand and the hydrophilic polysaccharide mixture on the other hand is 1:5.

In one embodiment, a single dosage unit of Terbutaline sulfate is about 4 to 8% by total weight.

The object of the present invention is further solved by a method for preparation of a controlled-release pharmaceutical composition comprising at least Terbutaline sulfate or a derivative thereof as an active agent, said method comprising the following steps:

(a) preparing a mixture comprising chitosan or a derivative thereof, xanthan gum or a derivative thereof, Terbutaline sulfate or a derivative thereof and optionally at least one additional pharmaceutically acceptable filler;

(b) compacting said mixture, preferably into a tablet.

In one embodiment, the tablet comprises from about 5 mg to about 7.5 mg Terbutaline sulfate.

In a preferred embodiment, the tablet comprises 5 mg Terbutaline sulfate.

The object of the present invention is further solved by a use of a controlled-release pharmaceutical composition comprising at least Terbutaline sulfate or a derivative thereof as an active agent for the manufacture of a medicament for inducing muscle relaxation in an animal, preferably a mammal, and most preferably human.

The object of the present invention is further solved by a method of treating illness of chronic nature in need of such treatment which comprises administering to the patient a therapeutically active agent that is basic in chemical nature or a salt of basic drug such as Terbutaline sulfate, the formula of prolonged release delivery contains xanthan gum and chitosan as a release retarding polymer carrier mixture and sodium bicarbonate as a buffering agent.

In one embodiment, the ratio of xanthan gum (anionic polymer) to chitosan (cationic polymer) 1:1 part by weight.

In one embodiment, the ratio of active agent and buffering agent (Terbutaline sultate and sodium bicarbonate) to polymer carrier mixture (chitosan and xanthan gum) ratio is 1:5.

In one embodiment, the controlled-release carrier consists essentially of a binary polymer mixture of chitosan and xanthan gum and sodium bicarbonate as a buffering system and release modifier as a direct compression formula.

In one embodiment, the tablet contains 5-7.5 mg Terbutaline sulfate.

In one embodiment, the controlled-release carrier is present in the range of 70-90%, specifically 83% by weight of the formulation.

In one embodiment, the controlled-release carrier is in the ratio of 1:1 by weight.

In one embodiment, the buffer system is a carbon dioxide generating and pH controlling system such as sodium bicarbonate and basic excipient that has a similar action.

In one embodiment, the buffer system is in the range of 5-20%, specifically 12.5% by weight.

The object of the present invention is further solved by a method of administrating Terbutaline sulfate or any basic drug and salt of a basic drug thereof in a controlled release dosage form to a patient in need thereof, said method comprising administration of solid unit dose of a therapeutic active agent of Terbutaline sulfate thereof and controlled-release carrier of xanthan gum and chitosan to an extent of at least 70% by weight of said carrier, wherein the controlled release carrier is present in a ratio of 1:1 parts by weight, the ratio of Terbutaline sulfate or any basic drug or salt of basic drug and the buffer system sodium bicarbonate to the controlled release carrier is 1:5.

In one embodiment, the controlled release solid dosage form is preferably formulated in a readily flowable direct compressible simple formula. However, granulation of such formula found not to affect its controlled release behavior.

In one embodiment, the controlled-release dosage form contains Terbutaline sulfate in the range of 4-8% by weight.

In one embodiment, the controlled-release solid dosage form is more stable under accelerated stability conditions in comparison with a commercial controlled-release product.

In one embodiment, the controlled release solid dosage form is obtained by simple mixing of the formula components and directly compress them using a suitable compaction machine.

The term "controlled-release" as used in the context of the present invention refers to a temporal and/or spatial control. "Temporal" can indicate a "sustained release" or any release altered or modulated with respect to time. Preferably, "controlled-release" refers to a "sustained release" or "prolonged release" or "release retardation". "Spatial" refers to any localized delivery.

An "active agent" in this context generally means a pharmacologically, therapeutically or otherwise effect agent which may have an effect itself or may become active, e.g. after being metabolized by endogenous enzymes or being converted under certain *in vivo* reaction conditions (e.g. at a certain pH).

According to the present invention, Terbutaline sulfate may be employed as any pharmaceutically acceptable salt thereof. Derivatives of Terbutaline are also considered.

An "inactive matrix" in this context means those components of the controlled-release pharmaceutical composition which serve as a carrier for the active agent without being itself pharmacologically, therapeutically or otherwise effective. Preferably, the active agent becomes mixed with the inactive matrix components during preparation of the controlled-release pharmaceutical composition such that the active agent is dispersed or otherwise embedded within the inactive matrix.

Apart from chitosan, also other hydrophilic polysaccharide polymers belonging to the group of chitin and salts and derivatives thereof may be considered.

When the term "% by weight" is used to indicate a percentage, this refers to the compostion's total weight, if not otherwise indicated.

A "ratio" as used herein generally refers to a ratio by weight, i.e. "w/w" and "w:w", respectively, if not otherwise indicated.

Preferably, controlled-release pharmaceutical compositions of the present invention are intended for the preparation of tablets generated by compaction, and preferably by direct compaction. The term "direct compaction" or "direct compression" means that all components constituting the controlled-release pharmaceutical composition are geometrically mixed before compression. Thus, compaction can be performed with powder. However, any processing, e.g. preparation of granules, which granules are subjected to compression, is also considered by the present invention. Furthermore, the use of the controlled-release pharmaceutical composition of the present invention for preparing other solid dosage forms, e.g. capsules, is also considered. Also taken into consideration is any refinement of the solid dosage form, e.g. coating of tablets. The controlled-release pharmaceutical composition of the present invention may also be incorporated within further delivery devices, e.g. a controlled-release plaster or a diaphragm pessary.

The capability of "pH controlling" means that a compound is able to maintain a certain pH, i.e. is a buffering agent.

One example of a "basic excipient" is sodium bicarbonate, which is used herein as a release modifying excipient.

A "controlled-release carrier" is a "controlled-release matrix".

In conclusion, the present invention provides for a controlled-release pharmaceutical composition having superior properties with respect to Terbutaline sulfate delivery compared to controlled-release compositions belonging to the state of the art.

## Detailed description of the invention

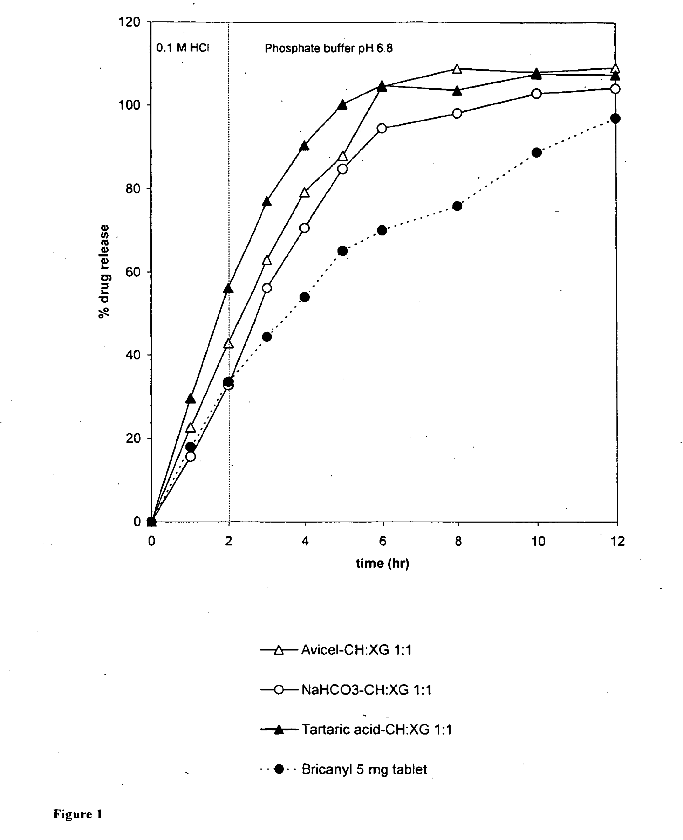

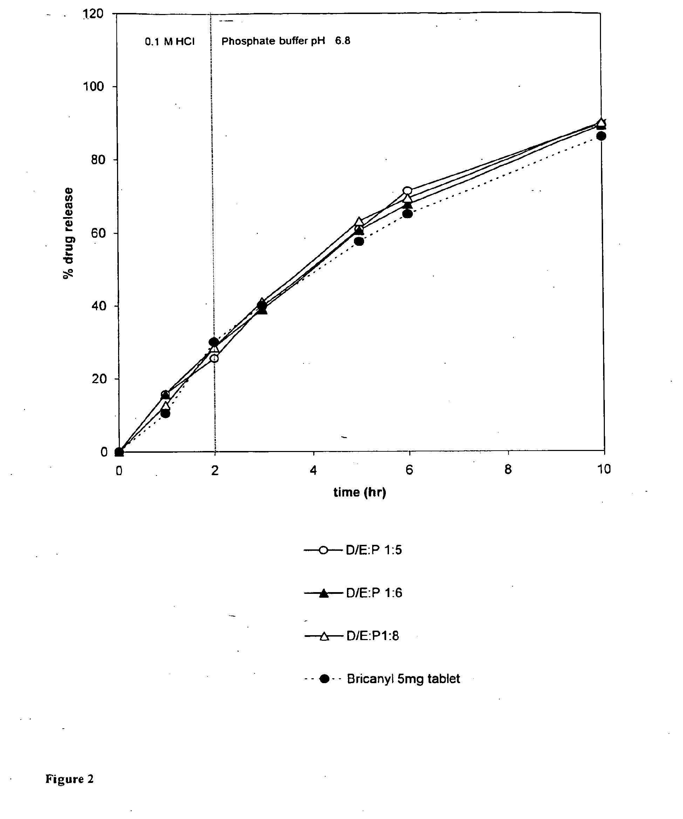

The invention shall now be further described and illustrated by the following examples with making reference to the attached Figures 1-29.

Figure 1    shows the time course of Terbutaline sulfate release from a controlled-release matrix of (CH:XG 1:1) comprising different excipients (D/E:P 1:3) compared to a Bricanyl 5 mg tablet.

Figure 2    shows the time course of Terbutaline sulfate release from a controlled-release matrix of (CH:XG 1:1) comprising $NaHCO_3$ as a release modifying excipient at different D/E:P ratios compared to a Bricanyl 5 mg tablet.

Figure 3    shows the time course of Terbutaline sulfate release from a controlled-release matrix of (CH:XG 1:1) at different D:P ratios compared to a Bricanyl 5 mg tablet.

Figure 4    shows the time course of Terbutaline sulfate release from controlled-release tablets (Batch S1, herein referred to as "Talin" or "Talin XR") compared to Bricanyl 5 mg tablets (Batch EA1536)

Figure 5    shows the dissolution profile obtained from HPLC analysis of Terbutaline sulfate in deionized water compared to that of Bricanyl Durules 5 mg.

Figure 6    shows the dissolution profile of Terbutaline sulfate obtained from HPLC analysis in 0.1 M HCl compared to that of Bricanyl Durules 5 mg.

Figure 7    shows the dissolution profile of Terbutaline sulfate obtained from HPLC analysis in phosphate buffer, pH 6.8 compared to that of Bricanyl Durules 5 mg.

Figure 8    shows the dissolution profile of Terbutaline sulfate obtained from UV spectrophotometric analysis in deionized water compared to that of Bricanyl Durules 5 mg.

Figure 9    shows DSC thermograms of sodium bicarbonate, Terbutaline sulfate and a physical mixture of Terbutaline sulfate and sodium bicarbonate 1:1 (w/w).

Figure 10   shows DSC thermograms of magnesium stearate, Terbutaline sulfate and a physical mixture of Terbutaline sulfate and magnesium stearate 1:1 (w/w).

Figure 11   shows DSC thermograms of xanthan gum, Terbutaline sulfate and a physical mixture of Terbutaline sulfate and xanthan gum 1:1 (w/w).

Figure 12   shows DSC thermograms of chitosan, Terbutaline sulfate and a physical mixture of Terbutaline sulfate and chitosan 1:1 (w/w).

Figure 13   shows the dissolution profile of Terbutaline sulfate from Bricanyl and Talin XR at room temperature.

Figure 14   shows the dissolution profile of Terbutaline sulfate from Bricanyl and Talin XR stored under the following conditions: 40°C/75% RH, closed, for 3 months.

Figure 15   shows a dissolution profile of Terbutaline sulfate from Bricanyl and Talin XR stored under the following conditions: 50°C, closed, for 3 months.

Figure 16   shows a dissolution profile of Terbutaline sulfate from Bricanyl and Talin XR stored under the following conditions: 40°C/75% RH, closed, for 6 months.

Figure 17   shows a dissolution profile of Terbutaline sulfate from Bricanyl and Talin XR under the following conditions: 40°C/75 RH, open, for 3 months.

Figure 18   shows the percent decrease in an assay of Talin XR and Bricanyl upon storage at 40°C/75% RH in closed bottles. Dashed line indicates the limits.

Figure 19   shows the percent decrease in an assay of Talin XR and Bricanyl upon storage at 40°C/ 75% RH in open bottles. Dashed line indicates the limits.

Figure 20   shows percent total impurities of Talin XR and Bricanyl upon storage at 40°C/75% RH in closed bottles (B.P. limits <0.4%).

Figure 21   shows percent total impurities of Talin XR and Bricanyl upon storage at 40°C/75% RH in open bottles (B.P. limits <0.4%).

Figure 22   shows percent individual impurities of Talin XR upon storage at 40°C/75% RH in closed bottles (B.P. limits <0.2%).

Figure 23   shows percent individual impurities of Bricanyl upon storage at 40°C/75% RH in open bottles (B.P. limits <0.2%).

Figure 24   shows percent individual impurities of Talin XR upon storage at 40°C/75% RH open bottles (B.P. limits <0.2%).

Figure 25   shows percent individual impurities of Bricanyl upon storage at 40°C/75% RH in open bottles (B.P. limits <0.2%).

Figure 26   shows a weight variation plot of 40 tablet representative samples of Talin XR BN 04.

Figure 27   shows a tablet hardness (N) plot of 20 tablet representative samples of Talin XR BN 04.

Figure 28   shows a content uniformity plot of 10 tablet representative samples of Talin XR BN 04.

Figure 29   shows a dissolution profile of Talin XR BN 04 compared to Bricanyl 5 mg tablets.

**Example 1:** Investigation of polymer combination controlled-release behavior

Preparation of a controlled-release tablet

The system contains 5 mg Terbutaline sulfate and 15 mg release modifing excipient (acidic: tartaric acid, or basic: sodium bicarbonate, or neutral: Avicel PH101) and 60 mg release retarding polymers per each single matrix. The polymer mixtures were CH:XG 1:1 (w/w). The drug/excipient to polymer mixture ratio was 1:3 (see Table 1). Tablets of 7 mm in diameter were prepared by a direct compression method. Components of each tablet were geometrically mixed by porcelain mortar and pestle for about 10 min before compression. Biplanar tablets were manufactured by compression of powder mixtures, applying a pressure of about 443 MPa for 15 sec by a hydraulic press.

*In vitro* dissolution test

The USP apparatus 1 (Basket) was used. The vessels were placed in a water bath regulated to maintain a temperature of 37 ± 0.5°C during the test. A fitted cover was used on the vessel to prevent any evaporation during the test. All tablets were subjected to 500 ml 0.1 M HCl USP solution for 2 hrs. The acidic medium was decanted and replaced with 500 ml phosphate buffer pH 6.8 USP solution for the rest of the dissolution time. The speed of dissolution test was set at 100 rpm. At specified time intervals, 6 ml aliquots were withdrawn. At each time interval, an aliquot equal in volume to the withdrawn sample was replaced to maintain the original volume of dissolution medium. The reported dissolution results were the average mean of three readings.

Samples of were taken and analyzed using an UV spectrophotometer. Absorbance data were measured at 235 nm, 2nd derivative. All calculations were performed with reference to the prepared calibration curves (see Table 2).

Table 1: Summary of different formulae used for the development of a Terbutaline sultate controlled-release product compared to a Bricanyl 5 mg tablet.

| Formula no. | 1 | 2 | 3 | 4* |
|---|---|---|---|---|
| Constituents (mg/tablet) | | | | |
| Terbutaline sulfate | 5 | 5 | 5 | 5 |
| Avicel PH 200 | 15 | 0 | 0 | 0 |
| Sodium bicarbonate | 0 | 15 | 0 | 0 |
| Tartaric acid | 0 | 0 | 15 | Yes |
| Chitosan | 30 | 30 | 30 | 0 |
| Xanthan gum | 30 | 30 | 30 | 0 |
| Total | 80 | 80 | 80 | 143 |

\* Reference commercial controlled-release product (Bricanyl Durules® 5 mg).

A Commercially available "Bricanyl 5 mg tablet" and "Bricanyl Durules 5 mg", respectively, (manufactured by AstraZeneca, Sweden; Lot no. EA1563, Man 2003-01, Exp 2006-01), was used as a reference. "Bricanyl® " and "Bricanyl Durules® " are registered trade marks of AstraZeneca. One tablet contains 5 mg Terbutaline sulfate and the following excipients: stearyl alcohol, ethyl cellulose, colloidal silicone dioxide, tartaric acid and polyvinyl chloride according to VIDAL.

Table 2: Summary of calibration curves of Terbutaline sulfate in 0.1 M HCl and in phosphate buffer, pH 6.8.

| Media | $\lambda_{max}$ (nm) 2nd derivative (non smooth) | Slope | Intercept | Conc. range (mg/100 ml) | R2 |
|---|---|---|---|---|---|
| 0.1 MHCl | 235 | 0.0023 | 2E-5 | 0.0811-5.00 | 0.9999 |
| Phosphate buffer, pH 6.8 | 235 | 0.0024 | 1E-6 | 0.04-2.50 | 0.9997 |

Comparison of drug release with a reference commercial product

The dissolution profile of a Bricanyl 5 mg tablet from AstraZeneca (Sweden) was used as a reference. The reference dissolution profile was compared with the samples via f2 factor of SUPAC (scale-up and postapproval change) suggested by FDA. Dissolution profiles of the reference samples would be considered similar when f2 is larger than 50:

$$F = 50 \cdot \log \left[ \frac{100}{\sqrt{1 + \frac{\sum_{t=1}^{t=n} [Rt - Tt]^2}{n}}} \right]$$

The use of CH/XG mixtures resulted in a better controlling release than the previous combinations (see Fig.

1), especially when a basic excipient (sodium bicarbonate) was used in the controlled-release formula. Based on calculations of similarity factor (f2), none of these formulae resulted in a release similar to Bricanyl, as shown in Table 3. But f2 indicated that formula 2 might be the best among these. Thus, further development should be conducted on formula 2. The only thing now to modify is the D/E:P ratio, it should be increased until a release behavior similar to Bricanyl is obtained.

Table 3: Similarity factor of calculations of the 6 formulae.

| Formula no. | Similarity factor (f2) |
|---|---|
| 1 | 32.77 |
| 2 | 40.51 |
| 3 | 28.17 |
| 4 | 30.00 |
| 5 | 34.64 |
| 6 | 26.29 |

Example 2: Screening out the suitable D/E:P ratio of the polymer mixture (P) (chitosan/xanthan 1:1) and the excipient (E) of sodium bicarbonate

The system contains 5 mg Terbutaline sulfate and 15 mg release modifying excipient (sodium bicarbonate) and release retarding polymer mixture. The polymer mixture was CH:XG in a ratio of 1:1. The drug/excipient to polymer mixture (D/E:P) ratio was 1:5, 1:6, and 1:8, as shown in Table 4. Tablets of 7 mm in diameter were prepared by a direct compression method. Components of each tablet were geometrically mixed by porcelain mortar and pestle for about 10 min before compression. Biplanar tablets were manufactured by compression of powder mixtures, applying a pressure of about 443 MPa for 15 sec by a hydraulic press. The *in vitro* dissolution conditions and the analysis methods were similar to that mentioned in Example 1.

Table 4: Summary of the formulae used for the development of a Terbutaline sulfate controlled-release product compared to a Bricanyl 5 mg tablet.

| Formula no. | 1 | 2 | 3 | 4 | 5 | 6* |
|---|---|---|---|---|---|---|
| Constituents (mg/tablet) | | | | | | |
| Terbutaline sulfate | 5 | 5 | 5 | 5 | 5 | 5 |
| Chitosan | 15 | 15 | 15 | 0 | 0 | |
| Xanthan gum | 50 | 60 | 80 | 37.5 | 75 | |
| Sodium bicarbonate | 50 | 60 | 80 | 37.5 | 75 | |
| Total | 120 | 140 | 180 | 80 | 155 | 144 |
| D:P ratio | | | | 1:15 | 1:30 | |
| D/E:P ratio | 1:5 | 1:6 | 1:8 | | | |

* Bricanyl Durules® 5 mg

The release from CH/XG matrix system was similar to that of Bricanyl in formulae 1, 2 and 3. Thus, increasing the D/E:P from 1:3 to 1:5 resulted in a release pattern much similar to that of Bricanyl. This is also demonstrated by the estimation of similarity factor f2, where 12 was higher than 50 in these formulae (see Table 5). Drug release from the Bricanyl depends on diffusion mechanism. Since the tablet stays intact with no increase in its dimension allover the process of dissolution, the surface area is constant during the process of dissolution. On the other hand, Talin XR tablet (for a definition, see Example 3 below) swells and the surface area is increasing with time in an acidic environment. In phosphate buffer, the surface area becomes constant and from which drug diffuses through. Also, a floating system is developed in the acidic medium due to the presence of sodium bicarbonate. This is beneficial since it will increase the tablet resident time in the gut.

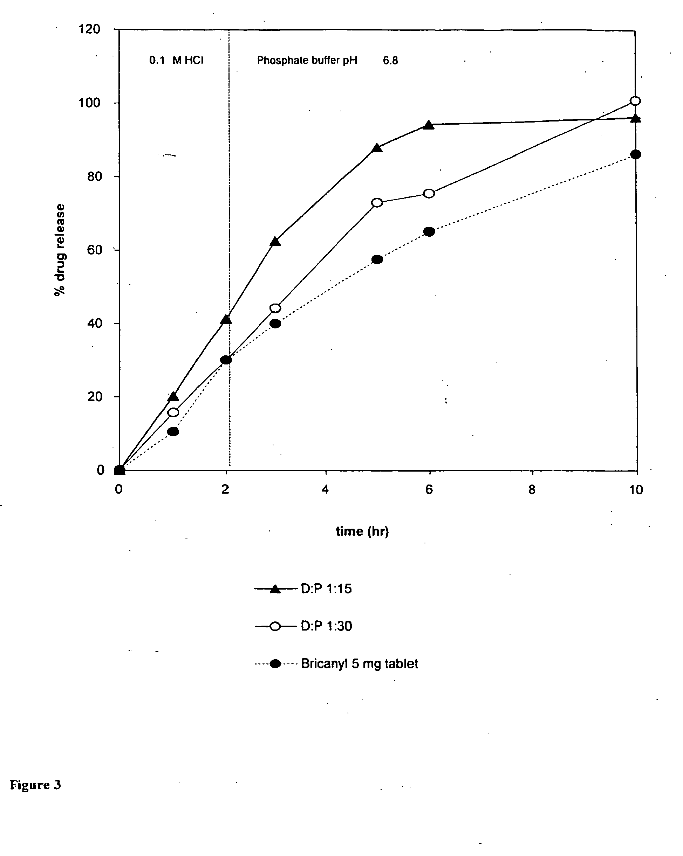

To illustrate the advantage of the use of sodium bicarbonate as a filler in CH/XG mixture, formulae 4 and 5 were investigated with high D:P ratios of 1:15 and 1:30, respectively. Sodium bicarbonate was not incorporated in these formulae. The drug release retardation power of these formulae was decreased tremendously although a very high concentration of the retarding polymer mixture was used (see Fig 3). The similarity factor 12 of formulae 4 and 5 was lower than 50, as shown in Table 5. This indicates that the release behavior was not similar to Bricanyl. Ultimately, the use of small concentrations of sodium bicarbonate increases the retardation power of CH/XG system and resulted in a system of similar drug release to Bricanyl.

Consequently, the cost effective formula that resulted in a release behavior similar to Bricanyl would be D/E: P 1:5. This formula must be enlarged for full investigation in small-scale experiment using a single punch machine. Then, if proven its quality, a scaling up program should be conducted.

Table 5: Similarity factor calculations of the 5 formulae.

| Formula no. | Similarity factor (f2) |
|---|---|
| 1 | 66.48 |
| 2 | 68.89 |
| 3 | 60.63 |
| 4 | 35.08 |
| 5 | 47.24 |

**Example 3:** Conduction of a small scale experiment through direct compression of the powder mixture D/E:P ratio 1: 5 using a single punch machine

The system contains 5 mg Terbutaline sulfate and 15 mg release modifying excipient (sodium bicarbonate) and release retarding polymer mixture per each tablet. The polymer mixture was CH:XG in a ratio of 1:1. The drug/ excipient to polymer mixture (D/E:P) ratio was 1:5 (see Table 6). Components of each tablet were geometrically mixed for about 10 min before compression. Biplanar tablets of 7 mm in diameter were prepared by direct compression using a single punch machine. The batch was 500g in size, and the batch number was referred to as S1. The proposed generic names for Batch S1 tablets is "Talin" and "Talin extended-release tablet" and "Talin XR". Five tablets were selected randomly from Bricanyl Durules and Talin XR for physical characterizations (see Tables 7 and 8, respectively). The *in vitro* dissolution conditions and the analysis methods were similar to those mentioned in Example 1.

Table 6: Summary of the formula (S1) used for the development of a Terbutaline sulfate controlled-release product (Talin XR) compared to a Bricanyl 5 mg tablet.

| Formula | |
|---|---|
| Constituents (mg/tablet) | |
| Terbutaline sulfate | 5 |
| Sodium bicarbonate | 15 |
| Xanthan gum | 49.5 |
| Chitosan | 49.5 |
| Magnesium Stearate | 1 |
| Total | 120 |
| D/E:P ratio | 1:5 |

Table 7: Physical properties of a Bricanyl 5 mg tablet (Batch EA1563).

| Tablet no. | 1 | 2 | 3 | 4 | 5 | Average | Standard deviation |
|---|---|---|---|---|---|---|---|
| Diameter (mm) | 7 | 7 | 7 | 7 - | 7 | 7 | 0 |
| Thickness (mm) | 2.7 | 2.7 | 2.7 | 2.7 | 2.7 | 2.7 | 0 |
| Weight (mg) | 144.30 | 141.30 | 143.70 | 143.70 | 143.00 | 143.20 | 1.158 |
| Hardness (N) | 71.00 | 63.00 | 62.00 | 65.00 | 67.00 | 65.60 | 3.578 |

Table 8: Physical properties of a Talin controlled-release tablet (Batch S 1).

| Tablet no. | 1 | 2 | 3 | 4 | 5 | Average. | Standard deviation |
|---|---|---|---|---|---|---|---|
| Diameter (mm) | 7 | | 7 | 7 | 7 | 7 | 0 |
| Thickness (mm) | 2.39 | 2.37 | 2.25 | 2.35 | 2.31 | 2.334 | 0.55 |
| Weight (mg) | 118.80 | 115.00 | 118.90 | 120.20 | 120.30 | 118.64 | 2.152 |
| Hardness (N) | 85.00 | 80.00 | 81.00 | 87.00 | 80.00 | 82.60 | 3.209 |

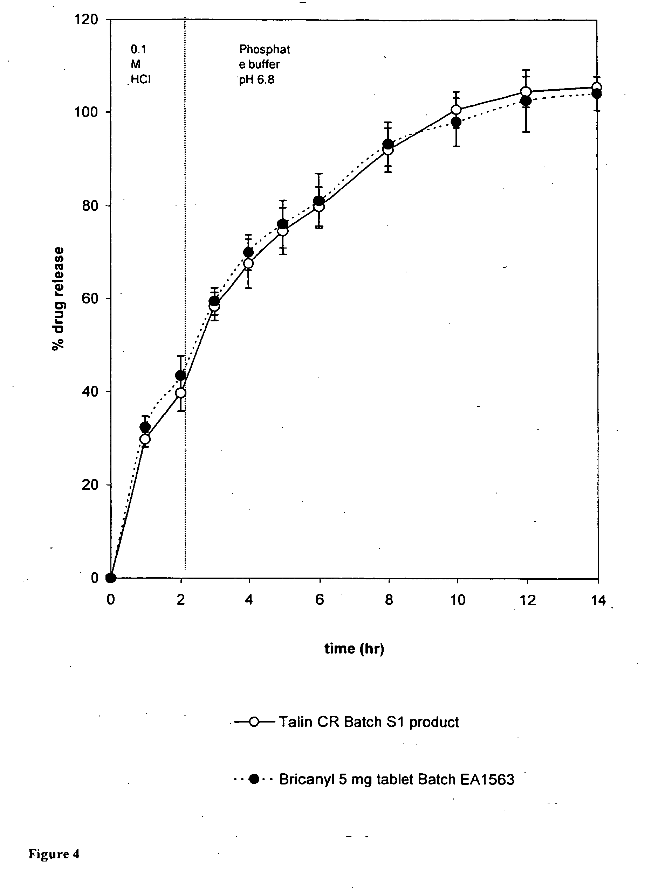

The release from Talin XR release matrix system (Batch S 1) was similar to that of Bricanyl, (see Fig. 4). Thus, the D/E:P 1:5 resulted in a release pattern much similar to that of Bricanyl Durules. This is also demonstrated by the estimation of similarity factor f2, where f2 was higher than 50 (around 80) in this formula (see Table 9).

The use of gradient dissolution media of two stages is an important criterion to judge Terbutaline sulfate release. The use of gradient *in vitro* dissolution media was found to be more correlated with the *in vivo* data than a single dissolution medium. The *in vivo/in vitro* correlation was established between the time for 80% of the drug release in gradient *in vitro* dissolution media and some *in vivo* parameters such as AUC and $C_{max}$ (D. Torres, G. Garcia-Encina, B. Seijo, and J. Vila-Jato, Biopharmaceutical Evaluation of Microencapsulated Ion-Exchange Resins Containing Diclofenac. Eur. J. Pharm. Biopharm. 41, 127-131, 1995; C. Liu, Y. Kao, S. Chen, T. Sokoloski, and M. Sheu, In-vitro Studies of Diclofenac Sodium Controlled-Release Matrix Tablets. J. Pharm. Pharmacol. 47, 360-364, 1995; A. Abu-Mahadi, In-vitro and In-vivo Evaluation of Modified Release Diclofenac Sodium Dosage Form (M.Sc. thesis). Supervised by Dr. Naji Najib and Dr. Khouloud Alkhamis. Submitted to the Jordan University of Science and Technology, Irbid, Jordan, in December 1999).

Ultimately, similarity between Talin XR and Bricanyl Durules using gradient pH dissolution was confirmed. However, similarity should be maintained if a single dissolution medium was applied, for example, in 0.1 M HCl, phosphate buffer, pH 6.8 and water. This also could be an important issue when postapproval changes are needed as pointed out in the Guidance of Industry of SUPAC-MR changes in the formulation.

Table 9: Determination of similarity factor f2 according to FDA regulations.

| Time (hrs) | Response (Reference product) | Response (Test product) | Difference | Difference 2 |
|---|---|---|---|---|
| 1 | 32.36 | 29.72 | 2.64 | 6.96 |
| 2 | 43.47 | 39.72 | 3.75 | 14.06 |
| 3 | 59.44 | 58.33 | 1.11 | 1.23 |
| 4 | 70.00 | 67.64 | 2.36 | 5.57 |
| 5 | 76.11 | 74.58 | 1.53 | 2.33 |
| 6 | 81.11 | 79.86 | 1.25 | 1.56 |
| 8 | 93.33 | 92.08 | 1.25 | 1.56 |
| 10 | 98.06 | 100.70 | -2.64 | 6.96 |
| 12 | 102.64 | 104.58 | -1.94 | 3.78 |
| 14 | 104.17 | 105.56 | -1.39 | 1.93 |
| | | | | |
| Sum (Difference 2) = | | | 45.97 | |
| | | | | |
| ((Sum(Difference2)/n)+1) 0.5 | | | 2.37 | |
| 100/(Sum(Difference^2)/n+1) 0.5 | | | 42.27 | |
| F=50*Log[100/Sum(Difference2)/n+1) 0.5)]= | | | 81.30 | |

**Example 4:** Similarity in release between Talin XR and Bricanyl Durules using different single dissolution media of water

The *in vitro* dissolution conditions were similar to those mentioned in Example 1. However, a single dissolution medium of water, 0.1 M HCl and phosphate buffer, pH 6.8 was used.

The details of the analysis conditions are shown in Table 10. The method of analysis used is validated internally. All calculations of HPLC analysis were performed for the average of two separate experiments. Standard samples were prepared according to the theoretical concentration, assuming full release of the active drug from the matrix into the dissolution medium.

Table 10: HPLC analysis conditions of dissolution sample.

| Active material | Terbutaline sulfate |
|---|---|
| Mobile phase (M.P.) | a mixture of 0.15 M ammonium acetate and glacial acetic acid (pH 4, 96:4, v/v); the mobile phase was filtered and degassed by sonication before use; |
| Flow rate (ml/min) | 2 ml/min |
| Loop volume ($\mu$l) | 100 $\mu$l |
| Column | Hypersil 100 ODS, C18, 5 $\mu$m, 10 cm *4.6 mm ID (Shandon, England) |
| Wave length (nm) | 270 nm |
| Final conc. standard (mg%) | 1.0 mg % |

Assay of Bricanyl and Talin XR

Twenty tablets were crushed using mortar and pestle. Accurately weighted quantities of powdered tablets equivalent to 15 mg Terbutaline sulfate were shaken with 100 ml water for 15 min and centrifuged. Two ml were taken and volume was completed to 25 ml with phosphate buffer, pH 6.8. The final concentration of the sample is around 1.2 mg/100 ml. The sample was analyzed using the HPLC method according to Table 10.

A standard was prepared of Terbutaline sulfate (75 mg) in 100 ml water. Five ml were taken and volume was completed to 25 ml with water. Four ml were taken and the volume was completed with phosphate buffer, pH 6.8. The final concentration of the standard is 1.2 mg/100 ml. The standard was analyzed using the HPLC method according to Table 10. Two repetitions were made for each sample and standard, and the mean was taken for statistical analysis.

In case of water, the hydrophilic polymers will dissolve with time until the tablet (Talin XR) has completely disappeared. This did not happen in the hydrophobic matrix of Bricanyl. It was intact all the time irrespective of the pH of the dissolution medium. Nevertheless, the similarity factor f2 showed that these two formulations showed similar release pattern (f2 >50).

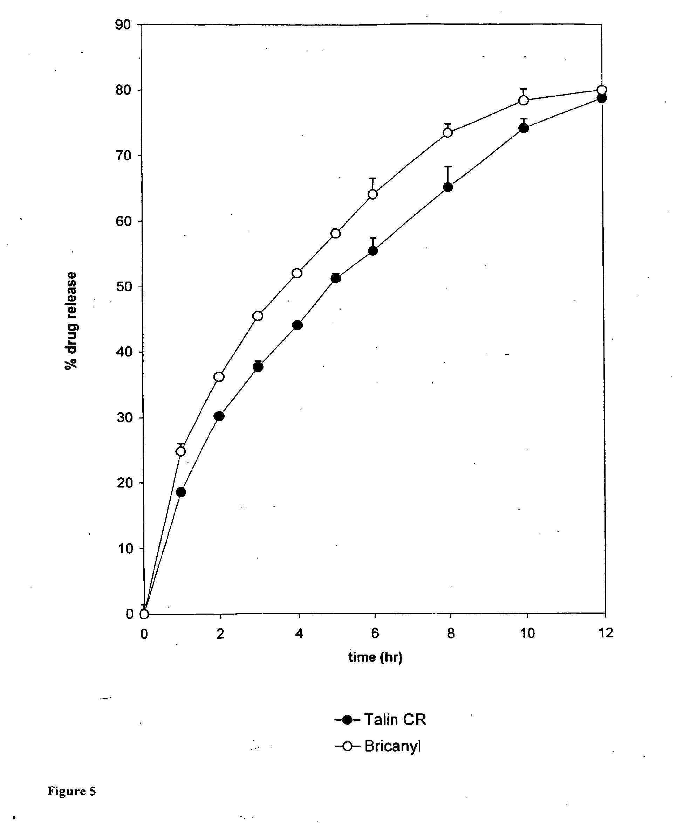

The dissolution profile analyzed on the basis of the HPLC method showed about 80% release after 12 hrs of dissolution (see Fig 5). The estimated f2 was higher than 50, as shown in Table 12. This indicates the similarity in release. Talin XR was similar in release to Bricanyl if water was used as a dissolution medium.

Table 11: Assay of Terbutaline sulfate in Talin XR and Bricanyl Durules initial condition.

| Sample | Condition | Weight of 5 units (mg) | Average weight unit (mg) | Sample weight (mg) | Final conc. (mg/100 ml) | Average response | % Assay Mean (CV%) |
|---|---|---|---|---|---|---|---|
| Talin CR | initial | 603 | 120.6 | 180.2, 180 | 1.201, 1.201 | 160223, 157673 | 97.4 (1.1) |
| Bricanyl | initial | 722 | 144.2 | 217, 217 | 1.202, 1.202 | 165992, 166573 | 101.3 (0.3) |

Table 12: Determination of similarity factor f2 according to FDA regulations obtained according to HPLC analysis.

| Time (hrs) | Response (Reference product) | Response (Test product) | Difference | Difference 2 |
|---|---|---|---|---|
| 1 | 24.80 | 18.60 | 6.20 | 38.44 |
| 2 | 36.23 | 30.22 | 6.01 | 36.08 |
| 3 | 45.52 | 37.72 | 7.80 | 60.85 |
| 4 | 52.05 | 44.13 | 7.93 | 62.81 |
| 5 | 58.10 | 51.22 | 6.88 | 47.31 |
| 6 | 64.10 | 55.45 | 8.65 | 74.82 |
| 8 | 73.40 | 65.10 | 8.30 | 68.89 |
| 10 | 78.36 | 74.15 | 4.21 | 17.68 |
| 12 | 79.90 | 78.70 | 1.20 | 1.44 |

(continued)

| Time (hrs) | Response (Reference product) | Response (Test product) | Difference | Difference 2 |
|---|---|---|---|---|
| Sum (Difference 2) = | | | | 408.32 |
| ((Sum(Difference2)/n)+1)0.5 | | | | 6.81 |
| 100/(Sum(Difference^2)/n+1)0.5 | | | | 14.69 |
| F=50*Log[100/(Sum(Difference2)/n+1) 0.5)] = | | | | 58.34 |

**Example 5:**

The objective of this example is to examine the similarity in release between Talin XR and Bricanyl Durules in three different single dissolution media (0.1 M HCl, water, phosphate buffer, pH 6.8).

The dissolution conditions and method of analysis are similar to that mentioned in Example 1 and Example 4, respectively.

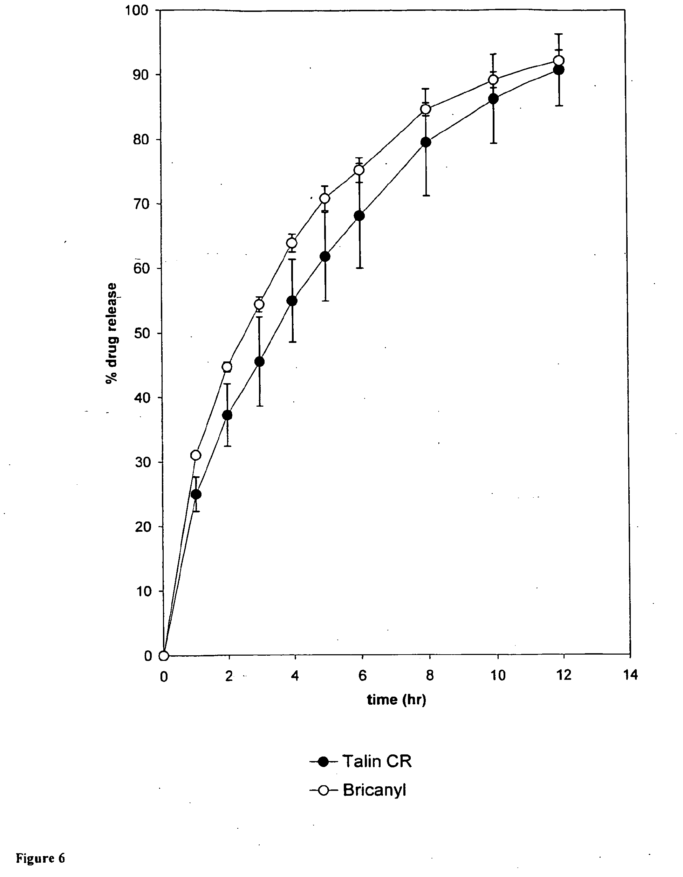

The dissolution profile in 0.1 M HCl medium of Talin and Bricanyl are shown in Fig. 6. The similarity factor was >50, as shown in Table 13. Thus, Talin XR is similar in release to Bricanyl if 0.1 M HCl was used as a dissolution medium.

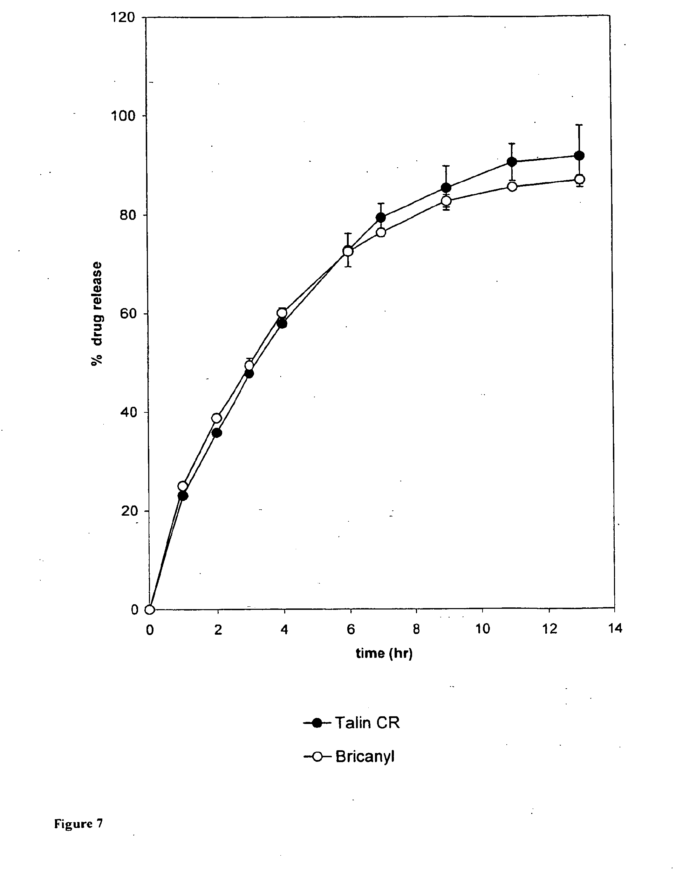

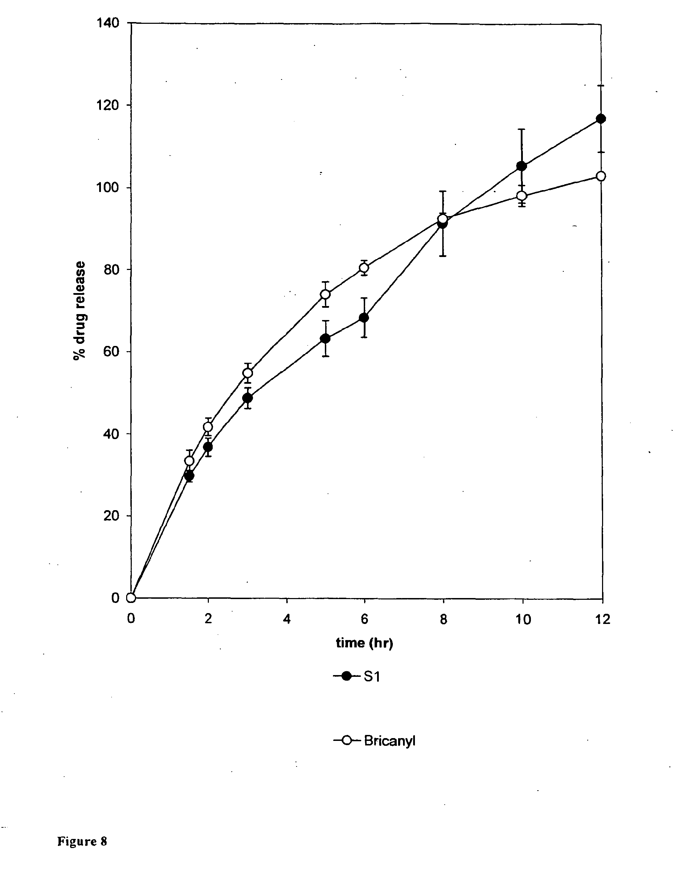

Figure 7 shows the dissolution profile of Talin XR and Bricanyl in phosphate buffer, pH 6.8. The dissolution profiles were similar, as indicated by the similarity factor (f2 >50; see Table 14). Figure 8 shows the dissolution profile of Talin XR and Bricanyl in water. The dissolution profiles were similar, as indicated by the similarity factor (f2 >50; see Table 15).

Table 13: Determination of similarity factor f2 according to FDA regulations obtained according to HPLC analysis.

| Time (hrs) | Response (Reference product) | Response (Test product) | Difference | Difference 2 |
|---|---|---|---|---|
| 1 | 31.05 | 25.01 | 6.04 | 36.42 |
| 2 | 44.76 | 37.26 | 7.50 | 56.18 |
| 3 | 54.50 | 45.60 | 8.90 | 79.21 |
| 4 | 63.92 | 55.01 | 8.91 | 79.36 |
| 5 | 70.85 | 61.85 | 9.00 | 81.00 |
| 6 | 75.25 | 68.15 | 7.10 | 50.41 |
| 8 | 84.69 | 79.55 | 5.14 | 26.42 |
| 10 | 89.15 | 86.25 | 2.90 | 8.41 |
| 12 | 92.20 | 90.70 | 1.50 | 2.25 |
| Sum (Difference 2) | | | 419.66 | |
| ((Sum(Difference2)/n)+1)0.5 | | | 6.90 | |
| 100/(Sum(Difference^2)/n+1)0.5 | | | 14.49 | |
| F=50*Log[100/(Sum(Difference2)/n+1)0.5)]= | | | 58.05 | |

Table 14: Determination of similarity factor f2 according to FDA regulations obtained according to HPLC analysis.

| Time (hrs) | Response (Reference product) | Response (Test product) | Difference | Difference 2 |
|---|---|---|---|---|
| 1 | 25.0625 | 23.0995 | 1.96 | 3.85 |
| 2 | 38.8195 | 35.815 | 3.00 | 9.03 |
| 3 | 49.4785 | 47.8625 | 1.62 | 2.61 |
| 4 | 60.0855 | 57.939 | 2.15 | 4.61 |
| 6 | 72.4345 | 72.715 | -0.28 | 0.08 |
| 7 | 76.29 | 79.302 | -3.01 | 9.07 |
| 9 | 82.6615 | 85.2795 | -2.62 | 6.85 |

(continued)

| Time (hrs) | Response (Reference product) | Response (Test product) | Difference | Difference 2 |
|---|---|---|---|---|
| 11 | 85.501 | 90.527 | -5.03 | 25.26 |
| 13 | 86.9055 | 91.6545 | -4.75 | 22.55 |

| | | | | |
|---|---|---|---|---|
| Sum (Difference 2) = | | | 61.36 | |
| ((Sum(Difference2)/n)+1) 0.5 | | | 2.80 | |
| 100/(Sum(Difference^2)/n+1) 0.5 | | | 35.76 | |
| F=50*Log[100/(Sum(Difference2)/n+1) 0.5)] = | | | 77.67 | |

Table 15: Determination of similarity factor f2 according to FDA regulations obtained according to HPLC analysis.

| Time (hrs) | Response (Reference product) | Response (Test product) | Difference | Difference 2 |
|---|---|---|---|---|
| 1 | 24.80 | 18.60 | 6.20 | 38.44 |
| 2 | 36.23 | 30.22 | 6.01 | 36.08 |
| 3 | 45.52 | 37.72 | 7.80 | 60.85 |
| 4 | 52.05 | 44.13 | 7.93 | 62.81 |
| 5 | 58.10 | 51.22 | 6.88 | 47.31 |
| 6 | 64.10 | 55.45 | 8.65 | 74.82 |
| 8 | 73.40 | 65.10 | 8.30 | 68.89 |
| 10 | 78.36 | 74.15 | 4.21 | 17.68 |
| 12 | 79.90 | 78.70 | 1.20 | 1.44 |

| | | | | |
|---|---|---|---|---|
| Sum (Difference 2) = | | | 408.32 | |
| ((Sum(Difference2)/n)+1) 0.5 | | | 6.80 | |
| 100/(Sum(Difference^2)/n+1) 0.5 | | | 14.69 | |
| F=50*Log[100/(Sum(Difference2)/n+1)0.5)] = | | | 58.34 | |

**Example 6:**

The objective of this study is to check excipient compatibility of the optimal formula using a DSC instrument.

Samples investigated by DSC are summarized in Table 16. Samples of 5-10 mg in weight were placed in hermetically sealed DSC pans. Samples were scanned at a rate of 10°C/min, and thermograms were obtained. The thermal analyzer (DSC) was calibrated prior to use by indium. The calibration procedure was repeated many times prior to DSC runs.

Table 16: Summary of DSC runs applied for excipient compatibility study.

| Substance / DSC run | Terbutaline sulfate % w/w | Xanthan gum % w/w | Chitosan | Magnesium stearate % w/w | NaHCO$_3$ % w/w |
|---|---|---|---|---|---|
| 1 | 100% | | | | |
| 2 | | 100% | | | |
| 3 | | | 100% | | |
| 4 | | | | 100% | |
| 5 | | | | | 100% |
| 6 | 50% | 50% | | | |
| 7 | 50% | | 50% | | |

(continued)

| Substance DSC run | Terbutaline sulfate % w/w | Xanthan gum % w/w | Chitosan | Magnesium stearate % w/w | NaHCO$_3$ % w/w |
|---|---|---|---|---|---|
| 8 | 50% | | | 50% | |
| 9 | 50% | | | | 50% |

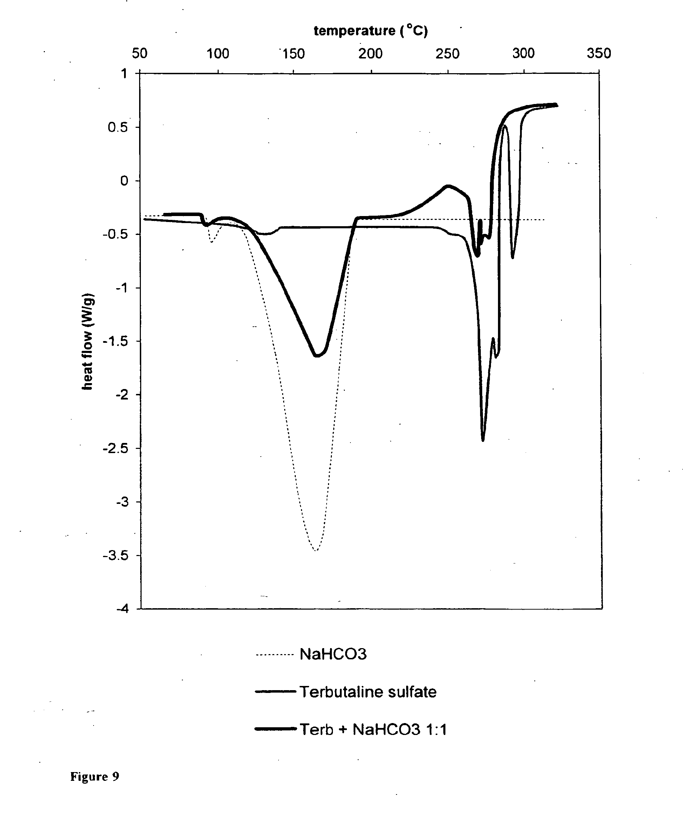

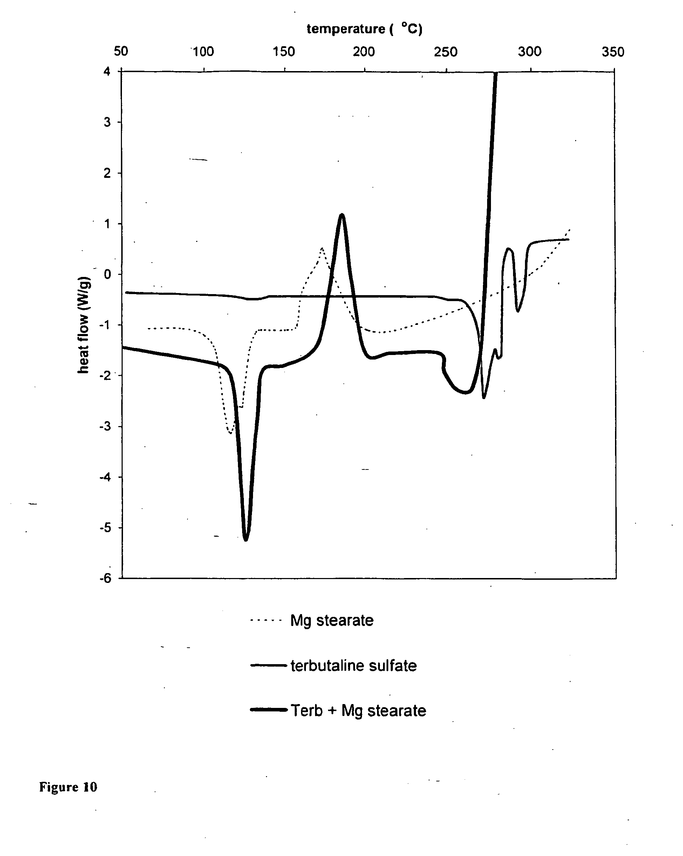

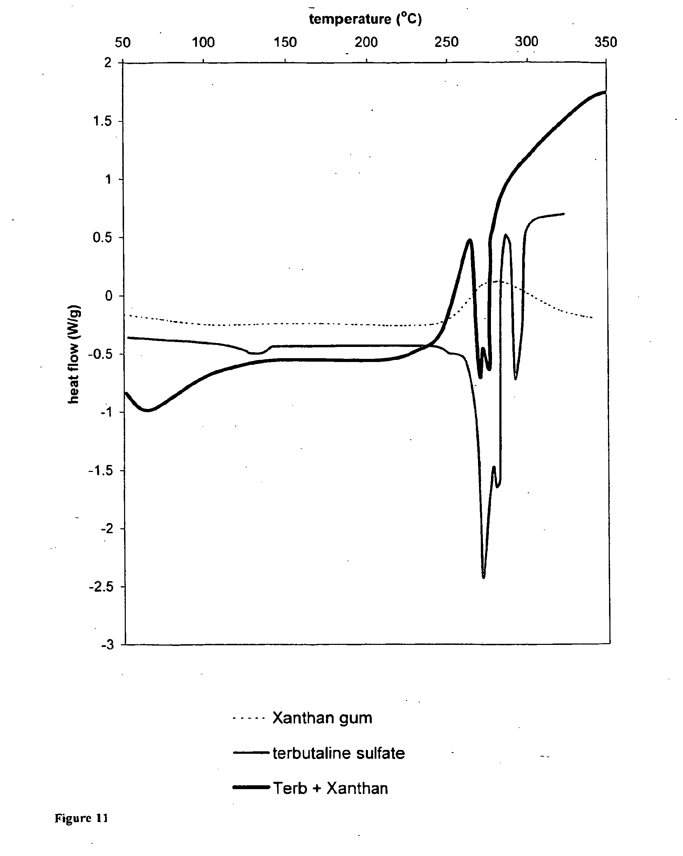

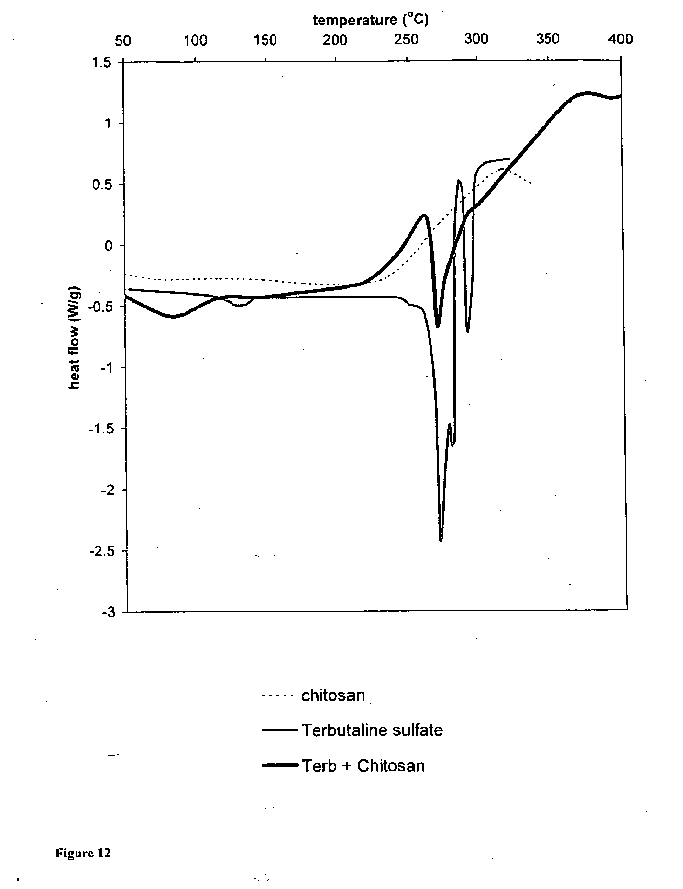

Figures 9-12 summarizes the DSC thermograms obtained from each excipient (release modifying or non-release modifying) and a mixture of each excipient with Terbutaline sulfate in a ratio of 1:1 (w/w). The resulted thermograms of the mixtures showed no odd behavior during the heat scan. These scans were almost equivalent to additive thermograms resulted between the drug and each excipient. This may indicate excipient compatibility with Terbutaline sulfate. Thus, it is concluded that excipients are compatible with Terbutaline sulfate according to DSC results.

**Example 7:**

The objective is an accelerated stability study of Talin XR compared to Bricanyl Durules under the storage conditions of 40°C/75% RH open, 40°C /75% RH closed, 50°C in terms of assay, related and dissolution profiles.

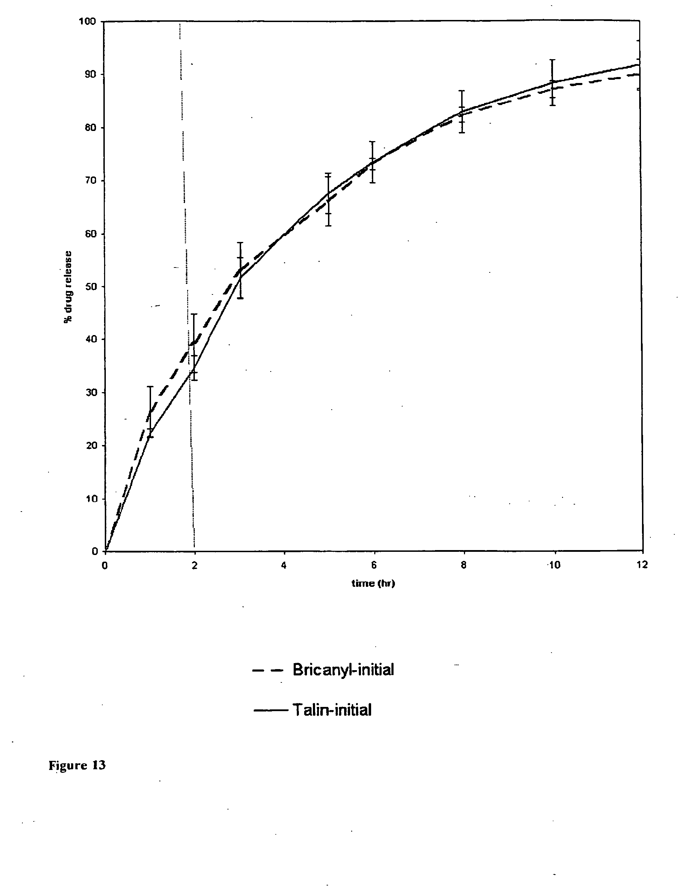

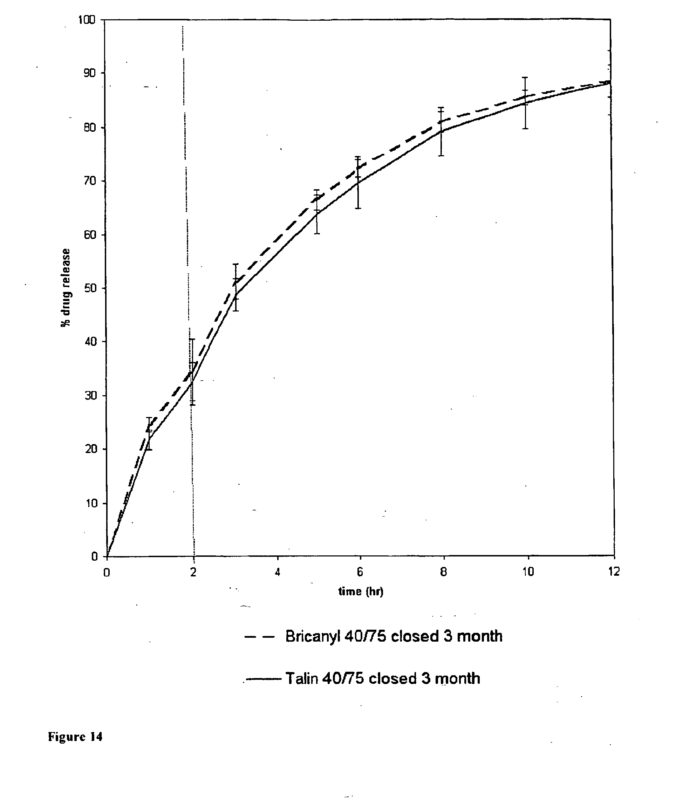

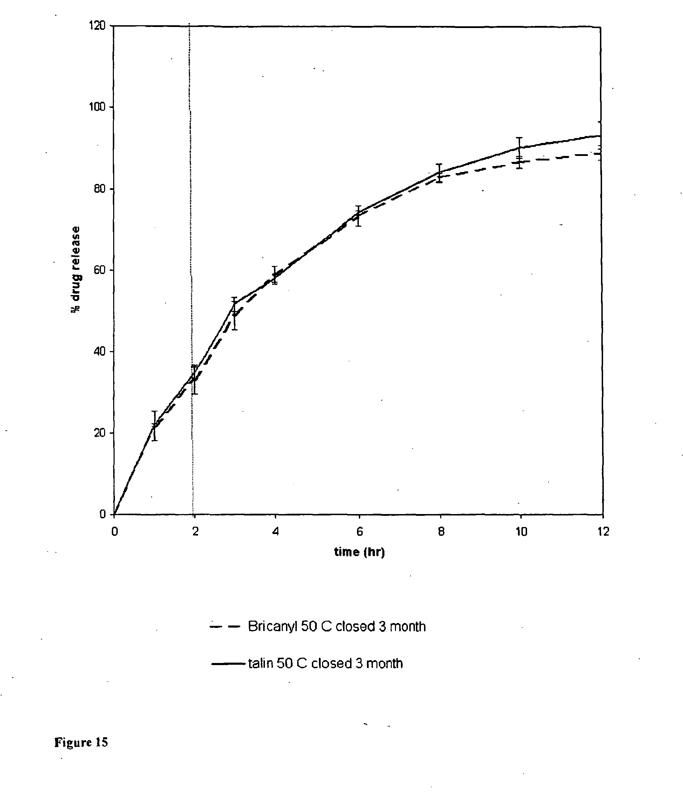

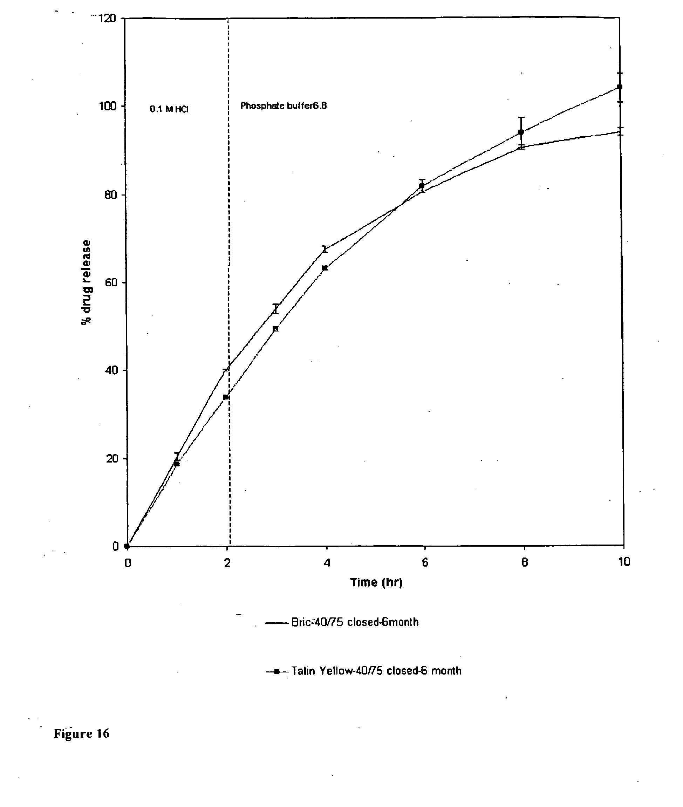

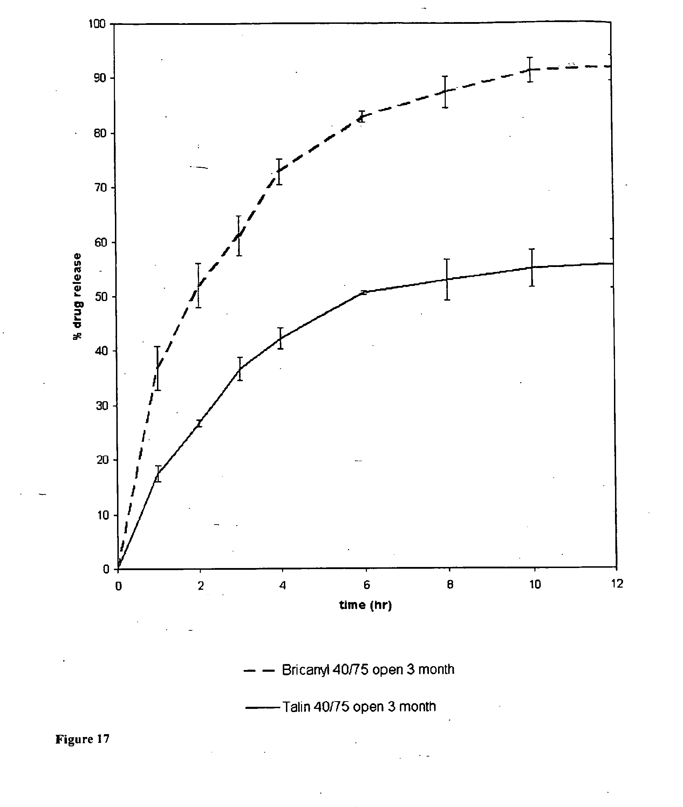

Talin XR and Bricanyl have shown similar *in vitro* dissolution release pattern under closed conditions, as shown in Figs 13-16. Similarity factors (f2) for each storage condition were higher than 50 for samples stored under closed conditions, Tables 17-20. This indicates the similarity in release behavior between Talin XR and Bricanyl 5 mg tablet under closed conditions. On the other hand, humid conditions showed large variations in release behavior (see Fig. 17). The release pattern was dissimilar, since f2 was <50 (see Table 21). Thus, Bricanyl and Talin XR tablets should be stored protected from humidity.

Table 17: Determination of similarity factor f2 according to FDA regulations at initial condition.

| Time (hrs) | Response (Reference product) | Response (Test product) | Difference | Difference 2 |
|---|---|---|---|---|
| 1 | 26.465 | 22.385 | 4.08 | 16.65 |
| 2 | 39.355 | 34.585 | 4.77 | 22.75 |
| 3 | 52.975 | 51.67 | 1.31 | 1.70 |
| 5 | 66.04 | 67.555 | -1.52 | 2.30 |
| 6 | 73.115 | 73.415 | -0.30 | 0.09 |
| 8 | 82.35 | 82.97 | -0.62 | 0.38 |
| 10 | 87.02 | 88.23 | -1.21 | 1.46 |
| 12 | 89.865 | 91.75 | -1.89 | 3.55 |

| | | |
|---|---|---|
| Sum (Difference 2 )= | | 48.89 |
| ((Sum(Difference2)/n)+1) 0.5 | | 2.67 |
| 100/(Sum(Difference^2)/n+1) 0.5 | | 37.50 |
| F=50*Log[100/(Sum(Difference2)/n+1) 0.5)] = | | 78.70 |

Table 18: Determination of similarity factor f2 according to FDA regulations at storage condition of 40°C/75% RH, closed, for 3 months.

| Time (hrs) | Response (Reference product) | Response (Test product) | Difference | Difference 2 |
|---|---|---|---|---|
| 1 | 24.515 | 21.72 | 2.80 | 7.81 |
| 2 | 34.385 | 32.528 | 1.86 | 3.45 |
| 3 | 51.14 | 48.733 | 2.41 | 5.79 |
| 5 | 66.41 | 63.723 | 2.69 | 7.22 |

(continued)

| Time (hrs) | Response (Reference product) | Response (Test product) | Difference | Difference 2 |
|---|---|---|---|---|
| 6 | 72.27 | 69.623 | 2.65 | 7.01 |
| 8 | 80.925 | 79.133 | 1.79 | 3.21 |
| 10 | 85.43 | 84.378 | 1.05 | 1.11 |
| 12 | 88.475 | 88.138 | 0.34 | 0.11 |

| | | | | |
|---|---|---|---|---|
| Sum (Difference 2) = | | | 35.71 | |

| | | | | |
|---|---|---|---|---|
| ((Sum(Difference2)/n)+1) 0.5 | | | 2.34 | |
| 100/(Sum(Difference^2)/n+1) 0.5 | | | 42.78 | |
| F=50*Log[100/(Sum(Difference2)/n+1)0:5)] = | | | 81.56 | |

Table 19: Determination of similarity factor f2 according to FDA regulations at storage condition of 50°C, closed, for 3 months.

| Time (hrs) | Response (Reference product) | Response (Test product) | Difference | Difference 2 |
|---|---|---|---|---|
| 1 | 21.7385 | 21.984 | -0.25 | 0.06 |
| 2 | 33.074 | 34.8905 | -1.82 | 3.30 |
| 3 | 48.8545 | 51.57 | -2.72 | 7.37 |
| 4 | 58.813 | 58.112 | 0.70 | 0.49 |
| 6 | 73.5365 | 74.2765 | -0.74 | 0.55 |
| 8 | 82.816 | 84.0835 | -1.27 | 1.61 |
| 10 | 86.5725 | 90.1365 | -3.56 | 12.70 |
| 12 | 89.0095 | 93.258 | -4.25 | 18.05 |

| | | | | |
|---|---|---|---|---|
| Sum (Difference 2 )= | | | 44.13 | |

| | | | | |
|---|---|---|---|---|
| ((Sum(Difference2)/n)+1 )0.5 | | | 2.55 | |
| 100/(Sum(Difference^2)/n+1) 0.5 | | | 39.17 | |
| F=50*Log[100/(Sum(Difference2)/n+1)0.5)] = | | | 79.65 | |

Table 20: Determination of similarity factor 12 according to FDA regulations at storage conditions of 40°C/75% RH, closed, for 6 months.

| Time (hrs) | Response (Reference product) | Response (Test product) | Difference | Difference 2 |
|---|---|---|---|---|
| 1 | 20.362 | 18.368 | -0.25 | 0.06 |
| 2 | 40.0745 | 32.0325 | -1.82 | 3.30 |
| 3 | 54.039 | 47.1565 | -2.72 | 7.37 |
| 4 | 67.5455 | 59.015 | 0.70 | 0.49 |
| 6 | 80.533 | 74.4095 | -0.74 | 0.55 |
| 8 | 90.545 | 84.56 | -1.27 | 1.61 |
| 10 | 94.129 | 92.4845 | -3.56 | 12.70 |

(continued)

| Time (hrs) | Response (Reference product) | Response (Test product) | Difference | Difference 2 |
|---|---|---|---|---|
| Sum (Difference 2 )= | | | 264.81 | |
| ((Sum(Difference2)/n)+1 )0.5 | | | 6.23 | |
| 100/(Sum(Difference^2)/n+1) 0.5 | | | 16.05 | |
| F=50*Log[100/(Sum(Difference2)/n+1)0.5)] = | | | 60.27 | |

Table 21: Determination of similarity factor 12 according to FDA regulations at storage conditions of 40°C/75 % RH, open, 3 months.

| Time (hrs) | Response (Reference product) | Response (Test product) | Difference | Difference 2 |
|---|---|---|---|---|
| 1 | 36.8125 | 17.5065 | 19.31 | 372.72 |
| 2 | 52.055 | 26.6785 | 25.38 | 643.97 |
| 3 | 61.116 | 36.719, | 24.40 | 595.21 |
| 4 | 72.8675 | 42.309 | 30.56 | 933.82 |
| 6 | 82.841 | 50.614 | 32.23 | 1038.58 |
| 8 | 87.3565 | 53.007 | 34.35 | 1179.89 |
| 10 | 91.184 | 54.9175 | 36.27 | 1315.26 |
| 12 | 91.7985 | 55.79 | 36.01 | 1296.61 |
| Sum (Difference 2 )= | | | 7376.06 | |
| ((Sum(Difference2)/n)+1 )0.5 | | | 30.38 | |
| 100/(Sum(Difference^2)/n+1) 0.5 | | | 3.29 | |
| F=50*Log[100/(Sum(Difference2)/n+1)0.5)] = | | | 25.87 | |

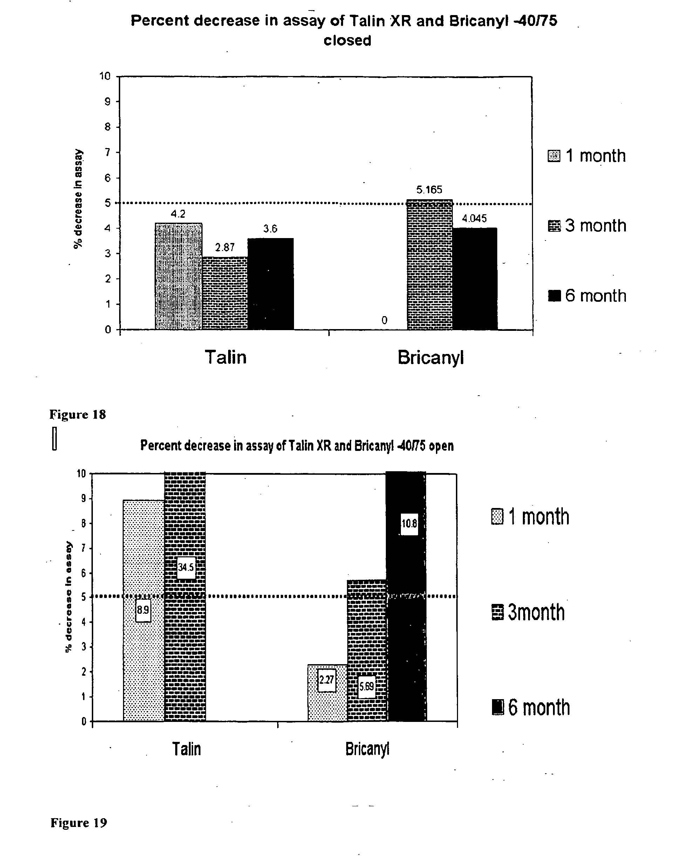

Table 22 summarizes the results of an assay for Talin XR and Bricanyl upon storage under different accelerated stability conditions analyzed after 1, 3 and 6 month periods. The percent decrease in the assay was less than 5% for samples stored under closed 40°C/75% RH conditions (see Fig 18). In contrast, samples stored under open conditions at 40°C/75% RH showed a high decrease of>5% (see Fig. 19). Talin XR showed also higher decrease in assay (>5%) upon storage under daylight (see Table 22). This result indicates that Talin XR and Bricanyl samples should be kept under closed conditions, i.e. protected from moisture. Talin XR should be protected from daylight.

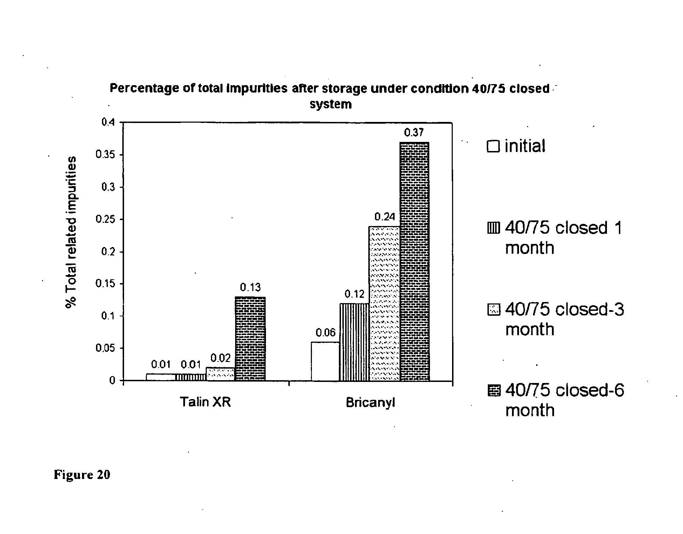

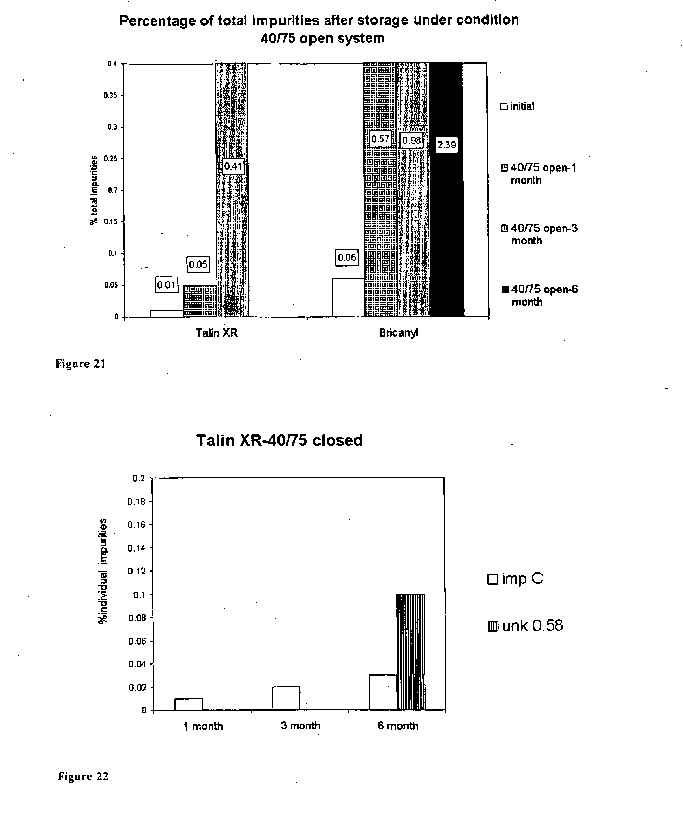

Table 23 summarizes the appearance of related impurities upon storage of Talin XR and Bricanyl under different accelerated stability conditions analyzed after 1, 3 and 6 month periods. The percent total impurities under closed conditions was less than 0.4 % (B.P. 2003 acceptance limit) for both Talin XR and Bricanyl, as shown in Fig. 20. However, under closed conditions both Talin XR and Bricanyl failed to pass the limit, as shown in Fig. 21. Thus, total impurity limit indicates that the products should be protected from moisture.

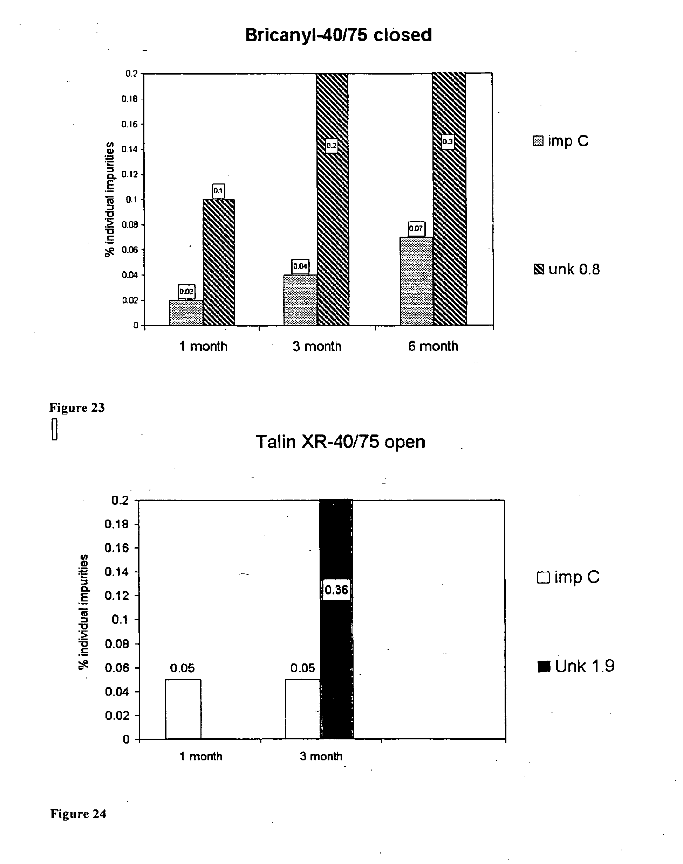

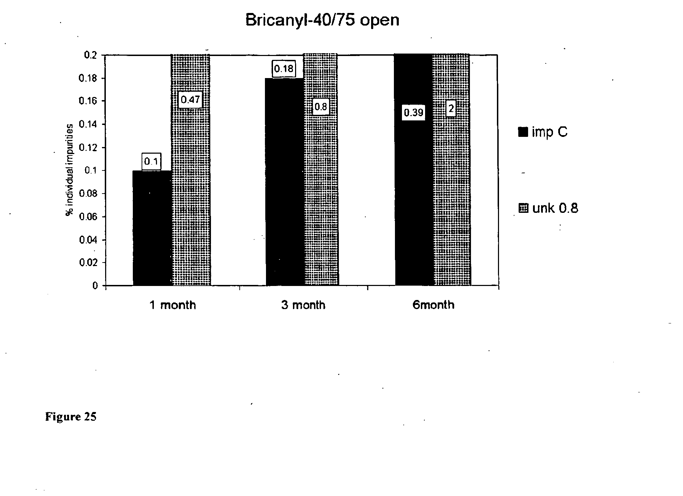

The individual impurity limit (stated to be less than 0.2% according to B.P. 2003) was less than 0.2 % for Talin XR and higher than 0.2 % for Bricanyl under closed 40°C/75%RH conditions (see Figs 22 and 3, respectively). This indicates the superiority of our formula of Talin XR over theBricanyl formula. Thus, Talin XR passes the test while Bricanyl fail to pass this pharmacopoeial test. On the other hand, under open conditions at 40°C/75 both Talin XR and Bricanyl failed to pass the individual impurity test (see Figs 24 and 25).

Table 24 summarizes different tests used to compare Bricanyl and Talin XR after 6-month storage. Accordingly, Talin XR provides a more stable formula than Bricanyl under closed storage condition at a temperature of 40°C.

Table 22: Summarizes the assay results of Talin and Bricanyl.

| Product | | | TalinXR | | | Bricanyl | | |
|---|---|---|---|---|---|---|---|---|
| Condition | period | average | CV% | %Drop | average | CV% | %Drop |
| initial | 0 | 97 | 1.9 | | 97.64 | 1.17 | 0 |
| 40/75 open | 1 month | 88.1 | | -8.9 | 95.37 | | -2.27 |
| | 3 months | 62.5 | 3.39 | -34.5 | 91.95 | 2.66 | -5.69 |
| | 6 months | | | | 86.8 | 4.24 | -10.8 |
| 40/75 closed | 1 month | 92.8 | | -4.2 | 99.8 | | 0 |
| | 3 months | 94.125 | 0.19 | -2.875 | 92.475 | 0.42 | -5.165 |
| | 6 months | 93.4 | 1.69 | -3.6 | 93.595 | 0.11 | -4.045 |
| 50 closed | 1 month | 91.7 | | -5.3 | | | |
| | 3 months | 96.69 | 0.13 | -0.31 | 98.75 | 0.93 | 0 |
| Daylight | 6 months | 89.405 | 2.3 | -7.595 | 99.03 | | 0 |

Table 23: Related impurities of Talin and Bricanyl.

| Product | | TalinXR | | | Bricanyl | | | |
|---|---|---|---|---|---|---|---|---|
| Condition | period | imp C | unk 1.9 | unk 0.58 | imp C | unk 0.28 | unk 0.58 | unk 0.8 |
| Initial | 0 | 0.01 | | | 0 | 0.01 | 0.05 | |
| 40/75 open | 1 month | 0.05 | | | 0.1 | | | 0.47 |
| | 3months | 0.05 | 0.36 | | 0.18 | | | 0.8 |
| | 6 months | | | | | | | |
| 40/75 closed | 1 month | 0.01 | | | 0.02 | | | 0.1 |
| | 3 months | 0.02 | | | 0.04 | | | 0.2 |
| | 6 months | 0.03 | | 0.1 | 0.07 | | | 0.3 |
| 50C closed | 1 month | 0.01 | | | | | | |
| | 3 months | 0.02 | | | | | | |
| Daylight | 6 months | 0 | 0 | | 0.14 | | 0.05 | |

Table 24: Summary of assay and related after 6 month storage.

| | Bricanyl | | | Talin XR | | |
|---|---|---|---|---|---|---|
| | 40/75 open | 40/75 closed | Daylight | 40/75 open | 40/75 closed | Daylight |
| % Dec Assay | (11%) fail | (4%) pass | (0%) pass | (>34%) fail | (3.6%) pass | (7.6%) fail |
| Related, individual* | (0.39%, 2%) fail | (0.3%) fail | (0.14%) pass | (>0.36) fail | (0.1%) pass | (0%) pass |
| Related, total | (2.4%) fail | (0.37%) pass | (0.19%) pass | (>0.41%) fail | (0.13%) pass | (0%) pass |
| Physical appearance | pass | pass | fail (brownish discoloration) | fail (black spots) | pass | pass |
| Dissolution profile similarity | | | | not similar | similar | similar |

(continued)

| | Bricanyl | | | Talin XR | | |
|---|---|---|---|---|---|---|
| | 40/75 open | 40/75 closed | Daylight | 40/75 open | 40/75 closed | Daylight |
| Net Result of each condition | fail | fail | fail | fail | pass | fail |

*The highest value of individual impurity was recorded

**Example 8:**

The objective of this study is the evaluation of Talin XR using a high speed tableting machine (Fette Tablet Press Machine P2100) of a SCALING UP (15 kg) Batch of Talin 5 XR tablets containing 5 mg Terbutaline sulfate.

Table 25 shows bulk density and sieve analysis of Talin 5 XR powder indicating the mixture particle size is <0.25 mm. This is useful in the case of these polymers since larger particle size was found to increase elastic recovery and decrease tablet hardness. Powder is flowable in tableting machine speed range and the tableting rate is >70,000 tablets/hr.

Table 25: Bulk density and sieve analysis of Talin 5 XR powder.

| Item | Result |
|---|---|
| Bulk density (g/cc) | 0.53 |
| Sieve analysis (%): | |
| 1.0mm | 0 |
| 0.71 min | 0 |
| 0.50 min | 0 |
| 0.355 mm | 1 |
| 0.25 mm | 1 |
| 0.18+0.09 mm | 83 |
| 0.06 mm | 13 |
| Fine | 2 |

Table 26 shows the uniformity of Terbutaline sulfate distribution in the mixture after 10 min mixing using the V-mixer. The results indicate the close proximity in content detection between UV and HPLC methods.

Table 26: Content uniformity of the powder mixture of Talin XR before compression.

| Sample site | Concentration (mg/100 ml) | Terbutaline sulfate content (UV spec method) (%) | Terbutaline sulfate content (HPLC method) (%) |
|---|---|---|---|
| Lower part | 7.01 | 100.38 | 101.65 |
| | 7.12 | 99.60 | 102.16 |
| Middle part | 7.06 | 100.48 | 99.69 |
| | 7.04 | 103.88 | 103.79 |
| Upper part | 7.10 | 104.65 | 106.19 |
| | 7.11 | 105.24 | 101.65 |

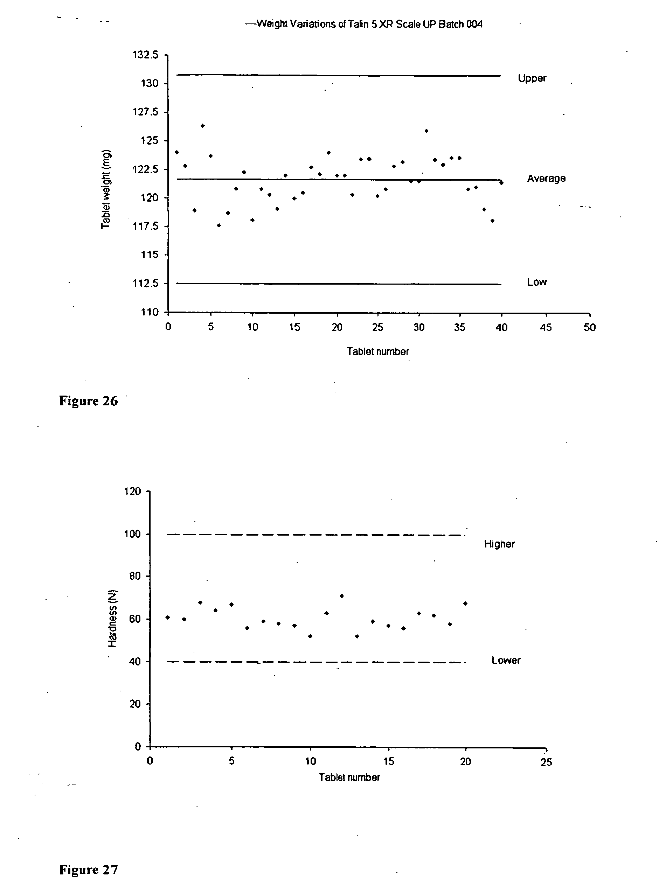

Figure 26 shows a weight variation plot of 40 tablets taken randomly from the batch (as a representative sample) of Talin XR. The plot indicates that the tablets' individual weights are within the acceptance limits of BP 2003 weight variation test.

Figure 27 shows tablet hardness (N) plot of 20 tablets (representative sample) of Talin XR. All tablet hardness fall within the proposed limits (40-100 N). The estimated friability of 20 tablets was 0.01 %. This is accepted since the limit is <1%.

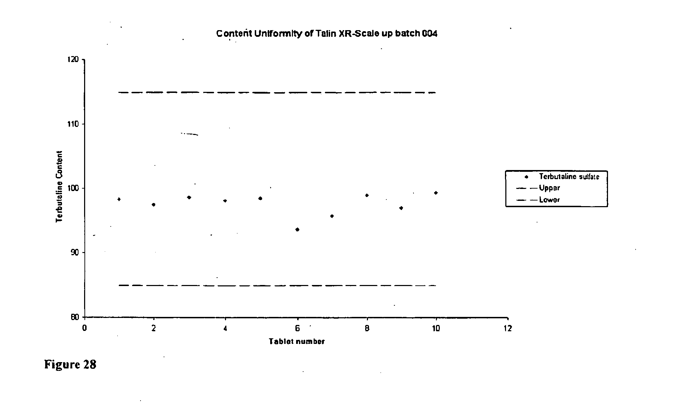

Figure 28 shows content uniformity of 10 tablets (representative sample) of Talin XR indicating that these tablets are within the accepted BP 2003 limits (85-115).

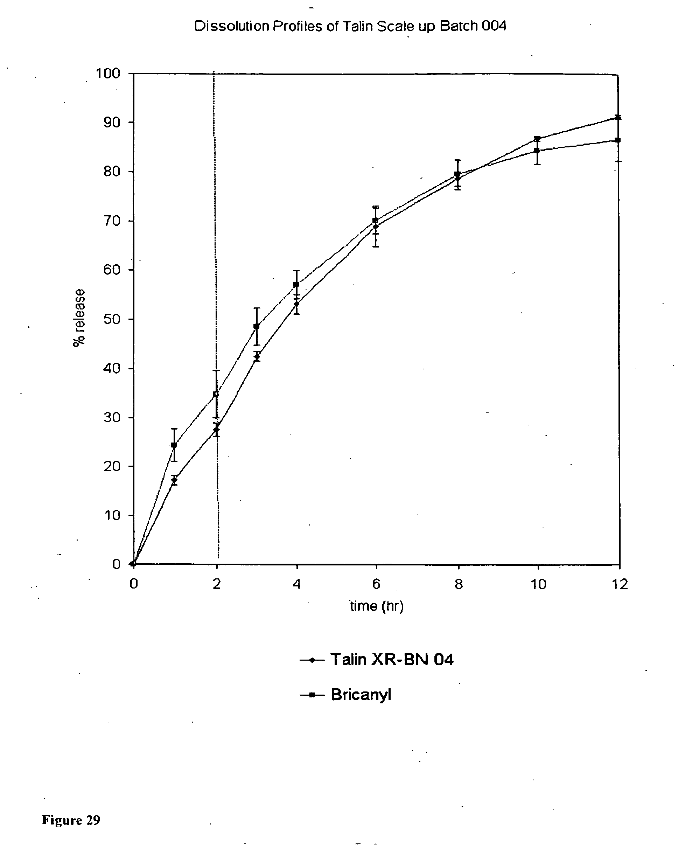

Figure 29 shows a dissolution profile of Talin XR compared to Bricanyl 5 mg tablets. The profile indicates the similarity in release with Bricanyl, as shown also in Table 27, where the similarity factor f2 >50.

An assay of Talin XR tablets taken from various sites in the batch indicates the homogeneity in the results of assay, as shown in Table 28. The assay was 100% using HPLC method and 98% in case of UV-method. This variation may be related to method of analysis. The sample passes the BP 2003 test of assay.

Three impurities appear unknown 1 (relative retention time RRT 0.6), unknown 2 (RRT 0.8) and impurity C (RRT 0.76). The % related in the sample was around 0.02% for each individual impurity. The BP 2003 limit for individual impurity is 0.2%. The total impurity limit should not be more than 0.4%. Consequently, the batch passes test of impurities the BP test. As a conclusion, this formula is applicable in the large-scale production machines.

Table 27: Determination of similarity factor f2 according to FDA regulations obtained according to HPLC analysis.

| Time (hrs) | Response (Reference product) | Response (Test product) | Difference | Difference 2 |
|---|---|---|---|---|
| 1 | 17.14 | 24.32 | -7.18 | 51.56 |
| 2 | 27.48 | 34.75 | -7.27 | 52.82 |
| 3 | 42.42 | 48.48 | -6.05 | 36.64 |
| 4 | 53.12 | 57.13 | -4.01 | 16.04 |
| 6 | 68.95 | 70.18 | -1.23 | 1.52 |
| 8 | 78.58 | 79.43 | -0.85 | 0.73 |
| 10 | 86.75 | 84.31 | 2.45 | 5.98 |
| 12 | 91.26 | 86.60 | 4.66 | 21.72 |

| | | |
|---|---|---|
| Sum (Difference 2) = | | 187.01 |
| ((Sum(Difference2)/n)+1) 0.5 | | 4.94 |
| 100/(Sum(Difference^2)/n+1) 0.5 | | 20.25 |
| F=50*Log[100/(Sum(Difference2)/n+1)0.5)] = | | 65.33 |

Table 28: Assay (initial) of Talin 5 XR

| Sample site | Final (mg/100 ml) | Conc % | |
|---|---|---|---|
| | | UV spec method | HPLC method |
| Sample 1-lower | 6.92 | 100.2278 | 104.2 |
| Sample 2-lower | 6.87 | 94.80033 | 98.9 |
| Sample 3-lower | 6.76 | 97.78688 | 101.6 |
| Sample 1- mid | 6.93 | 98.04861 | 99.9 |
| Sample 2-mid | 6.85 | 96.18543 | 98.6 |
| Sample 1-up | 6.91 | 96.05654 | 99.4 |
| Sample 2-up | 6.98 | 100.7258 | 99.5 |
| Average (%CV) | | 97.7 (2.3) | 100.3 (2.0) |

## Claims

1. A controlled-release pharmaceutical composition comprising at least Terbutaline sulfate or a derivative thereof as an active agent, and further comprising an inactive matrix, said matrix comprising a hydrophilic polysaccharide polymer mixture, said mixture comprising chitosan or a derivative thereof, and further comprising xanthan gum or a derivative thereof, wherein the ratio of xanthan gum and chitosan within said mixture is in the range from about 1:10 to about 10:1.

2. The controlled-release pharmaceutical composition according to claim 1, wherein the ratio of xanthan gum and chitosan is in the range from about 1:2 to about 2:1.

3. The controlled-release pharmaceutical composition according to claim 1 or 2, wherein the ratio of xanthan gum and chitosan is 1:1.

4. The controlled-release pharmaceutical composition according to any of the preceding claims, wherein the ratio of Terbutaline sulfate and the hydrophilic polysaccharide polymer mixture is in the range from about 1:1 to about 1:5, and preferably is 1:3.

5. The controlled-release pharmaceutical composition according to any of the preceding claims, optionally comprising at least one additional pharmaceutically acceptable filler.

6. The controlled-release pharmaceutical composition according to claim 5, wherein the pharmaceutically acceptable filler is selected from the group comprising calcium hydrogen phosphate, mannitol, Avicel PH 101, sodium bicarbonate and the like.

7. The controlled-release pharmaceutical composition according to claim 5 or 6, wherein the pharmaceutically acceptable filler is a carbon dioxide generating and/or pH controlling filler.

8. The controlled-release pharmaceutical composition according to any of claims 5 to 7, wherein the pharmaceutically acceptable filler is sodium bicarbonate.

9. The controlled-release pharmaceutical composition according to any of claims 5 to 8, wherein the percentage of the pharmaceutically acceptable filler is in the range from about 5% to about 20% by total weight, and preferably is 12.5% by total weight.

10. The controlled-release pharmaceutical composition according to any of claims 5 to 9 wherein the percentage of the pharmaceutically acceptable filler together with the hydrophilic polysaccharide polymer mixture is in the range from about 70 to about 90% by total weight, and preferably is 83 % by total weight.

11. The controlled-release pharmaceutical composition according to any of claims 5 to 10, wherein the ratio of Terbutaline sulfate and the pharmaceutically acceptable filler on the one hand and the hydrophilic polysaccharide mixture on the other hand is in the range of about 1:1 to about 1:5, and preferably is 1:5.

12. The controlled-release pharmaceutical composition according to any of the preceding claims, wherein a single dosage unit of Terbutaline sulfate is about 4 to 8% by total weight.

13. A method for preparation of a controlled-release pharmaceutical composition according to any of the preceding claims comprising at least Terbutaline sulfate or a derivative thereof as an active agent, said method comprising the following steps:

(a) preparing a mixture comprising chitosan or a derivative thereof, xanthan gum or a derivative thereof, Terbutaline sulfate or a derivative thereof and optionally at least one additional pharmaceutically acceptable filler;
(b) compacting said mixture, preferably into a tablet.

14. The method according to claim 13, wherein the tablet comprises from about 5 mg to about 7.5 mg Terbutaline sulfate, and preferably comprises 5 mg Terbutaline sulfate.

15. A use of a controlled-release pharmaceutical composition according to any of claims 1 to 12 comprising at least Terbutaline sulfate or a derivative thereof as an active agent for the manufacture of a medicament for inducing muscle relaxation in an animal, preferably a mammal, and most preferably human.

**Patentansprüche**

1. Pharmazeutische Zusammensetzung zur gesteuerten Freisetzung, welche mindestens Terbutalinsulfat oder ein Derivat davon als einen Wirkstoff umfasst, und die weiterhin eine inaktive Matrix umfasst, wobei die Matrix eine Polymerenmischung von hydrophilen Polysacchariden umfasst, wobei die Mischung Chitosan oder ein Derivat davon umfasst und weiterhin Xanthangummi oder ein Derivat davon umfasst, wobei das Verhältnis von Xanthangummi und Chitosan in der Mischung im Bereich von ungefähr 1:10 bis ungefähr 10:1 liegt.

2. Pharmazeutische Zusammensetzung zur gesteuerten Freisetzung nach Anspruch 1, wobei das Verhältnis von Xanthangummi und Chitosan im Bereich von ungefähr 1:2 bis ungefähr 2:1 liegt.

3. Pharmazeutische Zusammensetzung zur gesteuerten Freisetzung nach Anspruch 1 oder 2, wobei das Verhältnis von Xanthangummi und Chitosan 1:1 beträgt.

4. Pharmazeutische Zusammensetzung zur gesteuerten Freisetzung nach einem der vorangehenden Ansprüche, wobei das Verhältnis von Terbutalinsulfat und der Polymerenmischung von hydrophilen Polysacchariden im Bereich von ungefähr 1:1 bis ungefähr 1:5 liegt, und vorzugsweise 1:3 beträgt.

5. Pharmazeutische Zusammensetzung zur gesteuerten Freisetzung nach einem der vorangehenden Ansprüche, die wahlweise mindestens einen zusätzlichen pharmazeutisch annehmbaren Füllstoff umfasst.

6. Pharmazeutische Zusammensetzung zur gesteuerten Freisetzung nach Anspruch 5, wobei der pharmazeutisch annehmbare Füllstoff ausgewählt ist aus der Gruppe, die Calciumhydrogenphosphat, Mannitol, Avicel PH 101, Natriumbicarbonat und Ähnliches umfasst.

7. Pharmazeutische Zusammensetzung zur gesteuerten Freisetzung nach Anspruch 5 oder 6, wobei der pharmazeutisch annehmbare Füllstoff ein Kohlendioxid erzeugender und/oder den pH-Wert steuernder Füllstoff ist.

8. Pharmazeutische Zusammensetzung zur gesteuerten Freisetzung nach einem Ansprüche 5 bis 7, wobei der pharmazeutisch annehmbare Füllstoff Natriumbicarbonat ist.

9. Pharmazeutische Zusammensetzung zur gesteuerten Freisetzung nach einem der Ansprüche 5 bis 8, wobei der prozentuale Anteil des pharmazeutisch annehmbaren Füllstoffs im Bereich von ungefähr 5% bis ungefähr 20%, bezogen auf das Gesamtgewicht, liegt, und vorzugsweise 12,5%, bezogen auf das Gesamtgewicht, beträgt.

10. Pharmazeutische Zusammensetzung zur gesteuerten Freisetzung nach einem der Ansprüche 5 bis 9, wobei der prozentuale Anteil des pharmazeutisch annehmbaren Füllstoffs zusammen mit der Polymerenmischung von hydrophilen Polysacchariden im Bereich von ungefähr 70 bis ungefähr 90%, bezogen auf das Gesamtgewicht, liegt, und vorzugsweise 83%, bezogen auf das Gesamtgewicht, beträgt.

11. Pharmazeutische Zusammensetzung zur gesteuerten Freisetzung nach einem der Ansprüche 5 bis 10, wobei das Verhältnis von Terbutalinsulfat und dem pharmazeutisch annehmbaren Füllstoff einerseits und der hydrophilen Polysaccharidmischung andererseits im Bereich von ungefähr 1:1 bis ungefähr 1:5 liegt, und vorzugsweise 1:5 beträgt.

12. Pharmazeutische Zusammensetzung zur gesteuerten Freisetzung nach einem der vorangehenden Ansprüche, wobei eine einzelne Dosiereinheit von Terbutalinsulfat ungefähr 4 bis 8%, bezogen auf das Gesamtgewicht, beträgt.

13. Verfahren zum Herstellen einer pharmazeutischen Zusammensetzung zur gesteuerten Freisetzung nach einem der vorangehenden Ansprüche, welche mindestens Terbutalinsulfat oder ein Derivat davon als einen Wirkstoff umfasst, wobei das Verfahren die folgenden Schritte umfasst:

(a) Herstellen einer Mischung, welche Chitosan oder ein Derivat davon, Xanthangummi oder ein Derivat davon, Terbutalinsulfat oder ein Derivat davon und wahlweise mindestens einen zusätzlichen pharmazeutisch annehmbaren Füllstoff umfasst;
(b) Kompaktieren der Mischung, vorzugsweise zu einer Tablette.

14. Verfahren nach Anspruch 13, wobei die Tablette ungefähr 5 mg bis ungefähr 7,5 mg Terbutalinsulfat umfasst, und vorzugsweise 5 mg Terbutalinsulfat umfasst.

15. Verwendung einer pharmazeutischen Zusammensetzung zur gesteuerten Freisetzung nach einem der Ansprüche 1 bis 12, welche mindestens Terbutalinsulfat oder ein Derivat davon als einen Wirkstoff umfasst, zur Herstellung eines Medikaments zum Hervorrufen einer Muskelrelaxierung bei einem Tier, vorzugsweise einem Säugetier, und am meisten bevorzugt beim Menschen.

**Revendications**

1. Composition pharmaceutique à libération contrôlée comprenant au moins du sulfate de terbutaline ou un dérivé de celui-ci comme agent actif, et comprenant en outre une matrice inactive, ladite matrice comprenant un mélange de polymère de polysaccharide hydrophile, ledit mélange comprenant du chitosane ou un dérivé de celui-ci, et comprenant en outre de la gomme xanthique ou un dérivé de celle-ci, dans laquelle le rapport de la gomme xanthique et du chitosane au sein dudit mélange se situe dans la gamme allant d'environ 1:10 à environ 10:1.

2. Composition pharmaceutique à libération contrôlée selon la revendication 1, dans laquelle le rapport de la gomme xanthique et du chitosane se situe dans la gamme allant d'environ 1:2 à environ 2:1.

3. Composition pharmaceutique à libération contrôlée selon la revendication 1 ou 2, dans laquelle le rapport de la gomme xanthique et du chitosane est 1:1.

4. Composition pharmaceutique à libération contrôlée selon l'une quelconque des revendications précédentes, dans laquelle le rapport du sulfate de terbutaline et du mélange de polymère de polysaccharide hydrophile se situe dans la gamme allant d'environ 1:1 à environ 1:5, et de préférence est 1:3.

5. Composition pharmaceutique à libération contrôlée selon l'une quelconque des revendications précédentes, comprenant de manière optionnelle au moins une charge additionnelle acceptable pharmaceutiquement.

6. Composition pharmaceutique à libération contrôlée selon la revendication 5, dans laquelle la charge acceptable pharmaceutiquement est sélectionnée parmi le groupe comprenant le phosphate d'hydrogène de calcium, le mannitol, l'Avicel PH 101, le bicarbonate de sodium et analogues.

7. Composition pharmaceutique à libération contrôlée selon la revendication 5 ou 6, dans laquelle la charge acceptable pharmaceutiquement est une charge générant du dioxyde de carbone et/ou contrôlant le pH.

8. Composition pharmaceutique à libération contrôlée selon l'une quelconque des revendications 5 à 7, dans laquelle la charge acceptable pharmaceutiquement est le bicarbonate de sodium.

9. Composition pharmaceutique à libération contrôlée selon l'une quelconque des revendications 5 à 8, dans laquelle le pourcentage de charge acceptable pharmaceutiquement se situe dans la gamme allant d'environ 5 % à environ 20 % du poids total, et de préférence est de 12,5 % du poids total.

10. Composition pharmaceutique à libération contrôlée selon l'une quelconque des revendications 5 à 9 dans laquelle le pourcentage de charge acceptable pharmaceutiquement avec le mélange de polymère de polysaccharide hydrophile se situe dans la gamme allant d'environ 70 à environ 90 % du poids total, et de préférence est de 83 % du poids total.

11. Composition pharmaceutique à libération contrôlée selon l'une quelconque des revendications 5 à 10, dans laquelle le rapport du sulfate de terbutaline et de la charge acceptable pharmaceutiquement d'une part et du mélange de polysaccharide hydrophile d'autre part se situe dans la gamme allant d'environ 1:1 à environ 1:5, et de préférence est de 1:5.

12. Composition pharmaceutique à libération contrôlée selon l'une quelconque des revendications précédentes, dans laquelle une seule dose de sulfate de terbutaline est environ 4 à 8 % du poids total.

13. Méthode pour la préparation d'une composition pharmaceutique à libération contrôlée selon l'une quelconque des revendications précédentes comprenant au moins du sulfate de terbutaline ou un dérivé de celui-ci comme agent actif, ladite méthode comprenant les étapes suivantes :

la préparation d'un mélange comprenant du chitosane ou un dérivé de celui-ci, de la gomme xanthique ou un dérivé de celle-ci, du sulfate de terbutaline ou un dérivé de celui-ci et de manière optionnelle au moins une charge additionnelle acceptable pharmaceutiquement ;

le compactage dudit mélange, de préférence sous la forme d'une pilule.

14. Méthode selon la revendication 13, dans laquelle la pilule comprend d'environ 5 mg à environ 7,5 mg de sulfate de terbutaline, et de préférence comprend 5 mg de sulfate de terbutaline.

15. Utilisation d'une composition pharmaceutique à libération contrôlée selon l'une quelconque des revendications 1 à 12 comprenant au moins du sulfate de terbutaline ou un dérivé de celui-ci comme agent actif pour la fabrication d'un médicament pour induire une relaxation du muscle chez un animal, de préférence un mammifère, et de façon préférée entre toutes chez l'homme.

—△— Avicel-CH:XG 1:1

—○— NaHCO3-CH:XG 1:1

—▲— Tartaric acid-CH:XG 1:1

·· ●·· Bricanyl 5 mg tablet

Figure 1

Figure 2

Figure 3

Figure 4

Figure 5

Figure 6

Figure 7

Figure 8

Figure 9

temperature ( °C)

heat flow (W/g)

· · · · · Mg stearate

——— terbutaline sulfate

━━━ Terb + Mg stearate

Figure 10

temperature (°C)

----- Xanthan gum

——— terbutaline sulfate

——— Terb + Xanthan

**Figure 11**

temperature (°C)

heat flow (W/g)

····· chitosan

—— Terbutaline sulfate

—— Terb + Chitosan

**Figure 12**

- - Bricanyl-initial

—— Talin-initial

**Figure 13**

Figure 14

36

Figure 15

Figure 16

— — Bricanyl 40/75 open 3 month

———Talin 40/75 open 3 month

**Figure 17**

**Percent decrease in assay of Talin XR and Bricanyl -40/75 closed**

Figure 18

**Percent decrease in assay of Talin XR and Bricanyl -40/75 open**

Figure 19

**Percentage of total impurities after storage under condition 40/75 closed system**

Figure 20

## Percentage of total impurities after storage under condition 40/75 open system

Figure 21

## Talin XR-40/75 closed

Figure 22

## Bricanyl-40/75 closed

Figure 23

## Talin XR-40/75 open

Figure 24

## Bricanyl-40/75 open

Figure 25

—Weight Variations of Talin 5 XR Scale UP Batch 004

**Figure 26**

**Figure 27**

Content Uniformity of Talin XR-Scale up batch 004

Figure 28

Dissolution Profiles of Talin Scale up Batch 004

Figure 29